# EUROPEAN PATENT SPECIFICATION

(11) **EP 0 640 080 B1**
(45) Date of publication and mention of the grant of the patent: **22.10.1997**
(21) Application number: 93906123.0
(22) Date of filing: 26.02.1993
(51) Int. Cl.: C07D 403/10, A61K 31/40, C07D 409/10, C07D 409/14, C07D 405/14, C07D 471/04

(54) **SUBSTITUTED INDOLES AS ANGIOTENSIN II ANTAGONISTS**
SUBSTITUIERTE INDOLE ALS ANGIOTENSIN II ANTAGONISTEN
INDOLES SUBSTITUES UTILES COMME ANTAGONISTES DE L'ANGIOTENSINE II

(30) Priority: 13.05.1992 US 882390; 04.02.1993 US 4869
(43) Date of publication of application: 01.03.1995
(73) Proprietor: SYNTEX (U.S.A.) INC., Palo Alto California 94304 (US)
(72) Inventor: FISHER, Lawrence, E., Mountain View, CA 94040 (US); CLARKE, David, E., Mountain View, CA 94040 (US); JAHANGIR, Alam, San Jose, CA 95131 (US); CLARK, Robin, D., Palo Alto, CA 94306 (US)
(74) Representative: Kjellsaa-Berger, Hanny, Dr.
(86) International application number: US9301533
(87) International publication number: WO9323391

(56) References cited:
- EP-A- 0 400 835
- EP-A- 0 400 974
- EP-A- 0 497 516

## Description

### BACKGROUND OF THE INVENTION

### Field of the Invention

The present invention relates to substituted indole, azaindole and tetrahydro-1H-pyrrolo[2,3-c]pyridin-7-one compounds and derivatives thereof, useful as angiotensin II antagonists, particularly effective in the control of smooth and cardiac muscle contraction, especially for the treatment of cardiovascular disorders such as hypertension and congestive heart failure. Additionally, the compounds of the present invention are useful for the treatment of chronic renal failure and disorders of the alimentary tract, and have procognitive, anxiolytic and antidepressant activities.

### Related Disclosures

The renin-angiotensin system is a fundamental physiological mechanism for regulating blood pressure in mammals. Angiotensinogen is secreted into the bloodstream by the liver. Angiotensinogen is then cleaved by the protease renin to yield the decapeptide angiotensin I, which in turn is hydrolyzed by angiotensin converting enzyme (ACE) to the octapeptide angiotensin II. Angiotensin I is inactive in the cardiovascular system, but angiotensin II has numerous cardiovascular-renal activities. For example, it stimulates the adrenal cortex to secrete aldosterone, which causes the kidneys to retain sodium and water, increasing blood pressure. It causes vasoconstriction. It also facilitates neurotransmission in the sympathetic nervous system.

The effects of angiotensin II, such as arteriolar vasoconstriction, contraction of gastro-intestinal smooth muscle, aldosterone secretion, glycogenolysis, alteration of renal function and CNS effects, are mediated by the activation of specific angiotensin II receptors on smooth and cardiac muscle, adrenal medulla, brain, liver and kidney. Angiotensin II receptors are categorized into subtypes, for example the AT-1 and AT-2 subtypes. It is evident that disease states associated with activation of angiotensin II receptors could be usefully treated by compounds possessing angiotensin II antagonist activity.

Various angiotensin II receptor antagonists are known. See, for example, U.S. Patent Nos. 4,333,943, 4,880,804, 5,053,329, 5,124,335, and European Patents 0 245 637, 0 253 310, and 0 291 969, and also Wong et al., *Hypertension* 15:459 (1990), *J*. *Phamacol. Exp. Ther.* 256:211 (1990), and Chiu et al., *Biochem. Biophys. Res. Comm*. 165:196-203 (1989).

### SUMMARY OF THE INVENTION

A first aspect of the present invention relates to compounds of the Formula (I): 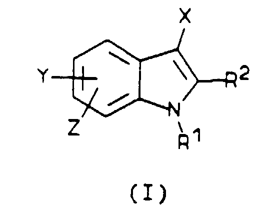 wherein :
R¹ is (C₁-C₁₀)alkyl, (C₃-C₈)cycloalkyl or (C₃-C₈)cycloalkyl (C₁-C₁₀)alkyl;
R² is 2"-(1H-tetrazol-5-yl)biphenyl-4'-ylmethyl;
X is hydrogen, (C₁-C₁₀)alkyl, halogen, -C(O)CF₃, -CO₂R⁴, or -C(O)NR⁵R⁶;
Y is hydrogen, (C₁-C₁₀)alkyl, (C₁-C₁₀)alkoxy, hydroxy, halogen, or -CO₂R⁴;
Z is hydrogen, (C₁-C₁₀)alkyl, (C₁-C₁₀)alkoxy, or halogen;
in which
R⁴ is hydrogen or (C₁-C₁₀)alkyl;
R⁵ is hydrogen, (C₁-C₁₀)alkyl, or -CH₂CO₂R⁴;
R⁶ is hydrogen or (C₁-C₁₀)alkyl; or
R⁵ and R⁶ taken together with the nitrogen to which they are attached represent a heterocycle selected from azetidine, azolidine, piperidine 4-methylpiperidine, hexamethyleneimine and heptamethyleneimine;
and the pharmaceutically acceptable salts thereof.

A second aspect of this invention relates to pharmaceutical compositions containing at least one compound of Formula (I) and one or more pharmaceutically acceptable excipients.

A third aspect of this invention relates to methods for treating angiotensin II receptor-related disorders in a mammal by administering an effective amount of a compound of Formula (I) or a composition containing a compound of Formula (I) to the mammal. In one embodiment, the angiotenein II receptor-related disorder treated is a cardiovascular disease, more particularly hypertension or congestive heart failure. In another embodiment, the angiotensin II receptor-related disorder treated is chronic renal failure or disorders of the alimentary tract. In yet another embodiment, the invention relates to methods for treating cognitive disorders, anxiety and depression.

A fourth aspect of the invention relates to methods for the preparation of the compounds of Formula (I).

A fifth aspect of the invention relates to intermediates of the Formula (1) shown below: 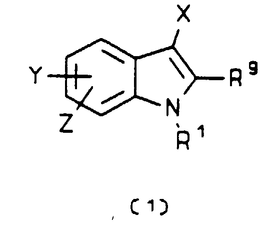 wherein X, Y, and Z are as defined above; R¹ is (C₁-C₁₀)alkyl, (C₃-C₈)cycloalkyl or (C₃-C₈)cycloalkyl(C₁-C₁₀)alkyl R⁹ is: 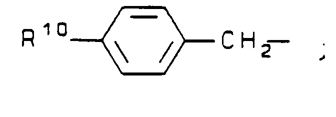 where R¹⁰ is:

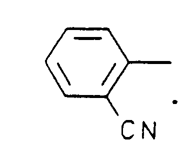

### DETAILED DESCRIPTION OF THE INVENTION

### Definitions

As used herein:

Alkyl saturated containing 1 to 10 carbon atoms are methyl, ethyl, propyl, isopropyl, tert-butyl, butyl, n-hexyl, n-decyl, and the like.

Cycloalkyl containing 3-8 carbon atoms are cyclopropyl, cyclobutyl, cyclopentyl, cyclohexyl, cycloheptyl and cyclooctyl.

(C₃-C₈)cycloalkyl (C₁-C₁₀)alkyl means cycloalkyl as defined attached to alkyl as defined, such as cyclopropylmethyl, cyclopentylmethyl, cyclohexylmethyl, 2-cyclohexylethyl, 2-cyclohexylpropyl, and the like.

The term "halo" means fluoro, bromo, chloro or iodo, unless otherwise indicated.

The terms "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform ("CHCl₃"), methylene chloride (or dichloromethane or "CH₂Cl₂"), diethyl ether, ethyl acetate, acetone, methylethyl ketone, methanol, ethanol, propanol, isopropanol, tert-butanol, dioxane, pyridine, and the like]. Unless specified to the contrary, the solvents used in the reactions of the present invention are inert solvents.

The compounds of Formula (I) form base addition salts by virtue of the presence of a 1H-tetrazol-5-yl group, and/or the presence of -CO₂H group. The term "pharmaceutically acceptable salt" means any salt derived from an inorganic or organic base chosen not to be biologically or otherwise undesirable. The term "pharmaceutically acceptable cation" means the cation of such base addition salts. The cations derived from inorganic bases include sodium, potassium, lithium, ammonium, calcium, magnesium and the like. Cations derived from organic bases include those formed from primary, secondary and tertiary amines, such as isopropylamine, diethylamine, trimethylamine, pyridine, cyclohexylamine, ethylene diamine, monoethanolamine, diethanolamine, triethanolamine and the like.

"Optional" or "optionally" means that the subsequently described event or circumstance may or may not occur, and that the description includes instances where said event or circumstance occurs and instances in which it does not. For example, "optionally followed by converting the free acid to the base addition salt" means that said conversion may or may not be carried out in order for the process described to fall within the invention, and the invention includes those processes wherein the free base is converted to the acid addition salt and those processes in which it is not.

The term "mammal" includes humans and all domestic and wild mammals, including, without limitation, cattle, horses, swine, sheep, goats, dogs, cats, rabbits, and the like.

The term "treatment" as used herein covers any treatment of a disease in a mammal, particularly a human, and includes:
(i) preventing the disease from occurring in a subject which may be predisposed to the disease but has not yet been diagnosed as having it;
(ii) inhibiting the disease, i.e. arresting its development; or
(iii) relieving the disease, i.e. causing regression of the disease.

The term "a disease-state that is alleviable by treatment with an angiotensin II antagonist" as used herein is intended to cover all disease states which are generally acknowledged in the art to be usefully treated with angiotensin II antagonists in general, and those disease states which have specifically been found to be usefully treated by the specific compounds of our invention, the compounds of Formula (I). Such disease states include, but are not limited to, cardiovascular disorders such as hypertension and congestive heart failure, chronic renal failure, cognitive and affective disorders, and disorders of the alimentary tract, for example inflammatory bowel disease.

The term "q.s." means adding a quantity sufficient to achieve a stated function, e.g., to bring a solution to the desired volume (i.e., 100%).

The term "effective amount" means a dosage sufficient to provide treatment for the disease state being treated. This will vary depending on the patient, the disease and the treatment being effected.

The naming and numbering of the compounds of the present invention are illustrated below.

The tetrazole group is illustrated with the hydrogen atom at the 1-position. 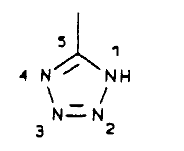 However, the tetrazole group is tautomeric, and consequently the hydrogen may be present at any of the 1-, 2-, 3- and 4-positions.

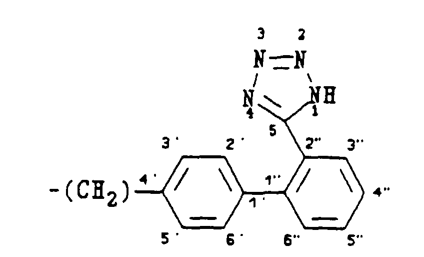

The above structure is named as 2"-(1H-tetrazol-5-yl)biphenyl-4'-ylmethyl.

A compound of Formula (I) is numbered as follows:

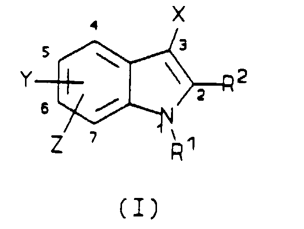

The compound of Formula (I) where X is -CO₂H, Y and Z are hydrogen, R¹ is n-butyl, and R² is 2"-(1H-tetrazol-5-yl)biphenyl-4'-ylmethyl is named:
1-(n-butyl)-2-[2"-(1H-tetrazol-5-yl)biphenyl-4'-ylmethyl]indole-3-carboxylic acid.

### Preferred Embodiments

Among the family of compounds of the present invention, one preferred category includes the compounds of Formula (I) where R¹ is 2"-(1H-tetrazol-5yl)biphenyl-4'-ylmethyl, X is -CO₂R⁴ and Z is hydrogen. Within this category one preferred group includes the compounds where R¹ is a branched or unbranched saturated hydrocarbon, especially where R⁴ is hydrogen, and more especially where R¹ is n-butyl. Another preferred group includes the compounds where R¹ is a branched or unbranched saturated hydrocarbon substituted by cycloalkyl, especially where R⁴ is hydrogen, and more especially where R¹ is cyclopropylmethyl or cyclohexylmethyl.

At present, the most preferred compounds are:
1-n-butyl-2-[2"-(1H-tetrazol-5-yl)biphenyl-4'-ylmethyl]-5-chloroindole-3-carboxylic acid;
1-n-butyl-2-[2"-(1H-tetrazol-5-yl)biphenyl-4'-ylmethyl]-3-trifluoroacetylindole; and
1-(n-butyl)-2-[2"-(1H-tetrazol-5-yl)biphenyl-4'-ylmethyl]-indole-3-carboxylic acid.

### PREPARATION OF COMPOUNDS OF FORMULA (I)

### I. Preparation of compounds of Formula (Ia)

The compounds of Formula (I) where R¹ is (C₁-C₁₀) alkyl, (C₃-C₈)cycloalkyl or (C₃-C₈)cycloalkyl (C₁-C₁₀)alkyl and R² is 2"- (1H-tetrazol-5-yl)biphenyl-4'-ylmethyl identified as Formula (Ia), may be prepared from the intermediates of Formula (11) or (12), the preparation of which is shown below in Reaction scheme I. 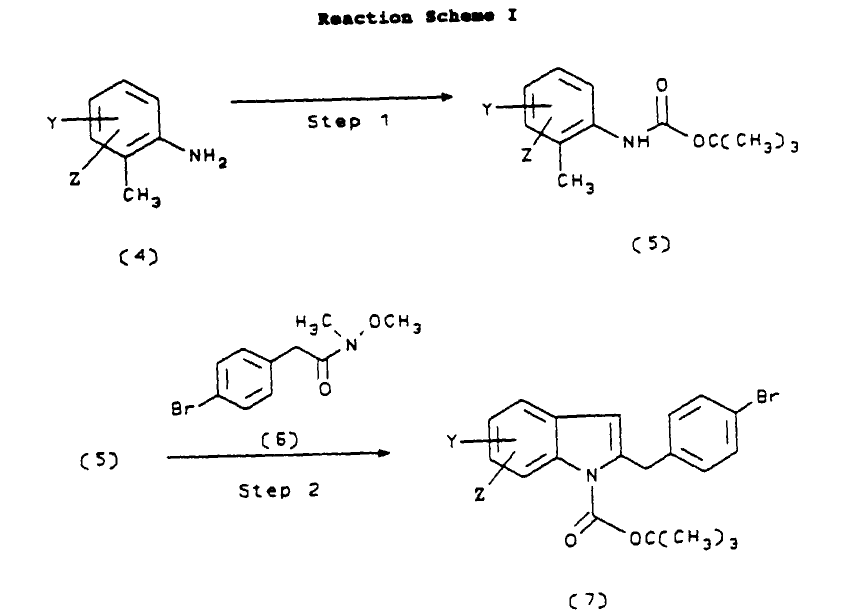 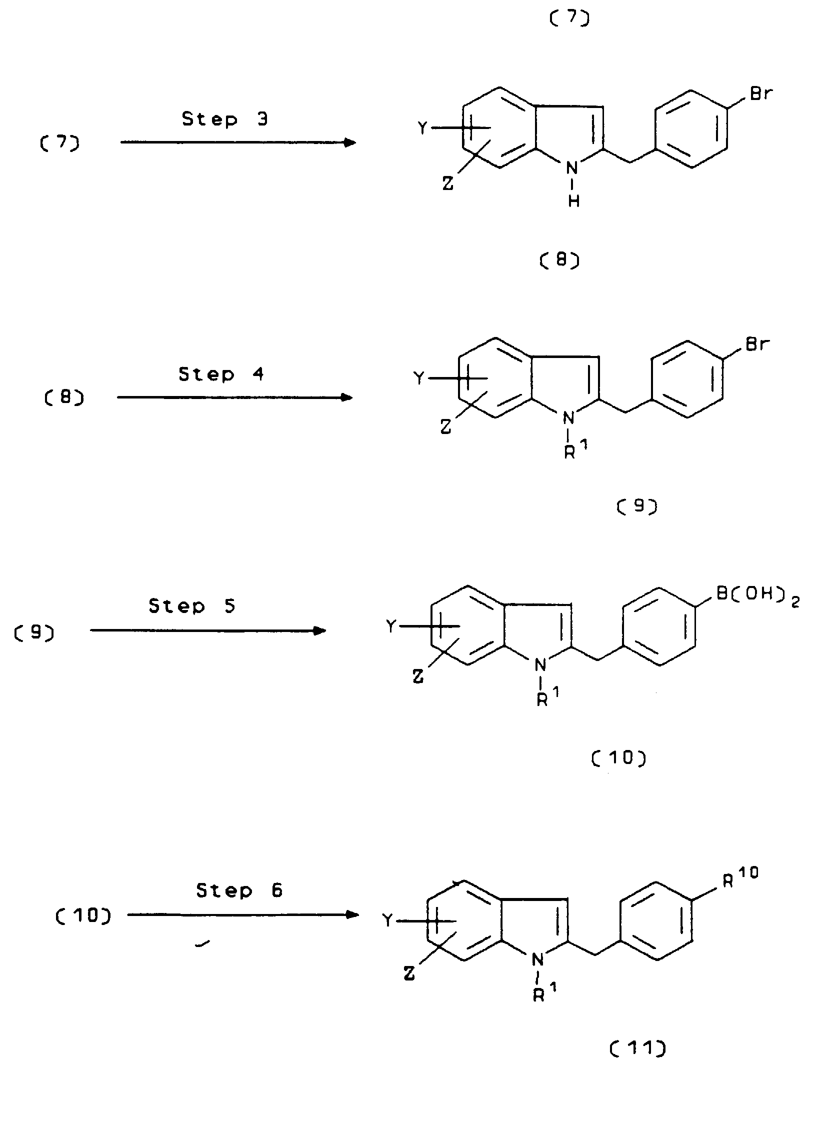 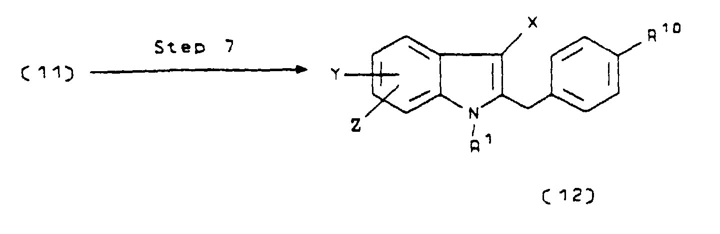 where R¹ is (C₁-C₁₀)alkyl, (C₃-C₈)cycloalkyl or (C₃-C₈)cycloalkyl(C₁-C₁₀)alkyl and R¹⁰, X, Y and Z are as defined in the Summary of the Invention.

### Starting Materials

The substituted aniline starting materials of Formula (4) are commercially available, for example from Aldrich Chemical Company. Alternatively, the substituted aniline compounds of Formula (4) can be prepared from commercially available substituted anilines by conventional means.

The reactant of Formula (6), N-methyl-N-methoxy-p-bromophenylacetylamide, used in step 2 of Reaction Scheme I, may be prepared according to the general method described in Nahm, S. and Weinreb, *S. Tet. Lett.* 22:3815-3818 (1981). Most preferably, this amide is prepared as follows:

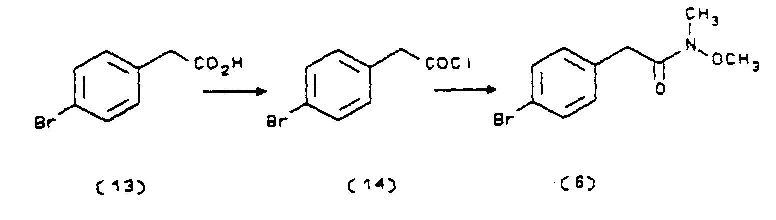

Step 1 of the preparation of N-methyl-N-methoxy-p-bromophenylacetylamide (6) uses p-bromophenylacetic acid, the starting material of Formula (13), which is commercially available, for example, from Aldrich Chemical Company.

Initially, oxalyl chloride is added to an equimolar amount of p-bromophenylacetic acid (13) in an inert solvent, preferably methylene chloride, at about room temperature, with a catalytic amount of dimethylformamide. The mixture is stirred for about 30 minutes, after which the product, p-bromophenylacetyl chloride (14), is isolated by conventional means.

Then p-bromophenylacetyl chloride (14) is dissolved in an inert solvent, preferably methylene chloride, cooled, and reacted with N-methyl-o-methylhydroxylamine hydrochloride in the same inert solvent, preferably methylene chloride, in the presence of a tertiary amine, preferably triethylamine. The product, N-methyl-N-methoxy-p-bromophenylacetylamide (6), is isolated by conventional means.

The 2-cyanobromobenzene used in step 6 of Reaction Scheme I is commercially available, i.a., from Aldrich.

The trialkyl tin azide used in step 8 is prepared as described in Kricheldorf and Leppert, *Synthesis,* pp 329-330 (1976).

### Preparation of Compounds of Formula (5)

To prepare a compound of Formula (5), a compound of Formula (4) is reacted with about 1-2 molar equivalents, preferably about 1 molar equivalent, of di-t-butyldicarbonate in an inert solvent (such as ether, dichloromethane, or tetrahydrofuran, preferably tetrahydrofuran). The reaction is carried out at a temperature of about 60°C to 100°C, preferably at about 80°C, for about 2 to 6 hours, preferably 4 hours. When the reaction is substantially complete the t-butyl-carbamate of Formula (5) is isolated by conventional means.

### Preparation of Compounds of Formula (7)

To prepare a compound of Formula (7), a compound of Formula (5) is first reacted with about 2 molar equivalents of a strong base (such as a butyllithium, preferably sec-butyllithium), in an ethereal solvent (such as diglyme, ether, or tetrahydrofuran, preferably tetrahydrofuran), at a temperature of about -30°C to -50°C, preferably at about -40°C. After addition of the base, the temperature is lowered to about -70°C to -100°C, preferably at about -75°C.

To the lithium dianion thus formed is added about 1 to 1.5 molar equivalents, preferably about 1 molar equivalent, of the compound of Formula (6) (N-methyl-N-methoxy-p-bromophenylacetylamide) in an ethereal solvent, preferably tetrahydrofuran, maintaining the temperature at about -80°C to - 50°C, preferably at about -60°C, for about 5 minutes to 1 hour, preferably about 10 minutes. When the reaction is substantially complete an uncyclized intermediate is isolated by conventional means, and dissolved in an inert aprotic solvent (such as ether, tetrahydrofuran, or methylene chloride; preferably methylene chloride), and about 0.1 to 2 molar equivalents, preferably about 1 molar equivalent, of trifluoroacetic acid is added. The reaction is carried out at about room temperature, for about 5 minutes to 1 hour, preferably about 30 minutes. When the reaction is substantially complete the t-butylcarboxyindole of Formula (7) is isolated by conventional means.

### Preparation of Compounds of Formula (8)

To prepare a compound of Formula (8), a compound of Formula (7) is reacted with an excess of a base (such as potassium hydroxide, ammonium hydroxide, or sodium hydroxide; preferably sodium hydroxide) in water and an organic solvent (such as methanol, propanol, or ethanol; preferably ethanol), at a temperature of about 50°C to reflux, preferably at about reflux, for 4 to 10 hours, preferably about 6 hours. When the reaction is substantially complete the 2-(p-bromophenyl-methyl)indole of Formula (8) is isolated by conventional means.

### Preparation of Compounds of Formula (9) where R¹ is (C₁-C₁₀)alkyl, (C₃-C₈)cycloalkyl or (C₃-C₈)cycloalkyl (C₁-C₁₀)alkyl

To prepare a compound of Formula (9) where R¹ is (C₁-C₁₀)alkyl, (C₃-C₈)cycloalkyl or (C₃-C₈)cycloalkyl (C₁-C₁₀)alkyl a compound of Formula (8) is first reacted with 1 to 1.5 molar equivalents, preferably about 1.1 molar equivalents, of an alkali metal hydride (such as lithium hydride, potassium hydride, or sodium hydride; preferably sodium hydride) in a polar solvent (such as acetamide, DMSO (dimethyl sulfoxide), or DMF (dimethylformamide); preferably DMF). The reaction is carried out at a temperature of -10°C to 20°C, preferably about 0°C, for about 5 minutes to 1 hour, preferably about 10 minutes. The anion thus formed is then reacted with about 1 to 1.5 molar equivalents, preferably about 1 molar equivalent, of a (C₁-C₁₀)alkyl, (C₃-C₈)cycloalkyl or (C₃-C₈)cycloalkyl (C₁-C₁₀)alkyl halide, preferably a (C₁-C₁₀)alkyl, (C₃-C₈)cycloalkyl or (C₃-C₈)cycloalkyl (C₁-C₁₀)alkyl iodide, for about 15 to 60 minutes, preferably about 30 minutes. When the reaction is substantially complete the 1-(C₁-C₁₀)alkyl, (C₃-C₈)cycloalkyl or (C₃-C₈)cycloalkyl (C₁-C₁₀)alkyl-2-(p-bromophenylmethyl)indole of Formula (9) where R¹ is (C₁-C₁₀)alkyl, (C₃-C₈)cycloalkyl or (C₃-C₈)cycloalkyl (C₁-C₁₀)alkyl is isolated by conventional means.

### Preparation of Compounds of Formula (10) where R¹ is (C₁-C₁₀)alkyl, (C₃-C₈)cycloalkyl or (C₃-C₈)cycloalkyl (C₁-C₁₀)alkyl

To prepare a compound of Formula (10) where R¹ is (C₁-C₁₀)alkyl, (C₃-C₈)cycloalkyl or (C₃-C₈)cycloalkyl (C₁-C₁₀)alkyl, a compound of Formula (9) where R¹ is (C₁-C₁₀)alkyl, (C₃-C₈)cycloalkyl or (C₃-C₈)cycloalkyl (C₁-C₁₀)alkyl is first reacted with 1 to 1.5 molar equivalents, preferably about 1.1 molar equivalents, of an alkyl lithium base, preferably n-butyllithium, in an ethereal solvent (such as ether, monoglyme, or tetrahydrofuran; preferably tetrahydrofuran). The reaction is carried out at a temperature of -100°C to -50°C, preferably about -70°C, for about 5 minutes to 1 hour, preferably about 15 minutes. The anion thus formed is then reacted with about 1 to 2 molar equivalents, preferably about 1.5 molar equivalents, of an alkyl borate (such as triethyl borate, trimethyl borate, or tributyl borate, preferably tributyl borate), allowing the reaction mixture to warm to about 0°C. When the reaction is substantially complete the 2-[4'-(dihydroxyboron)phenylmethyl]-indole of Formula (10) where R¹ is (C₁-C₁₀)alkyl, (C₃-C₈)cycloalkyl or (C₃-C₈)cycloalkyl (C₁-C₁₀)alkyl is isolated by conventional means.

### Preparation of Compounds of Formula (11) where R¹ is (C₁-C₁₀)alkyl, (C₃-C₈)cycloalkyl or (C₃-C₈)cycloalkyl (C₁-C₁₀)alkyl

To prepare a compound of Formula (11) where R¹ is (C₁-C₁₀)alkyl, (C₃-C₈)cycloalkyl or (C₃-C₈)cycloalkyl (C₁-C₁₀)alkyl, a compound of Formula (10) where R¹ is (C₁-C₁₀)alkyl, (C₃-C₈)cycloalkyl or (C₃-C₈)cycloalkyl (C₁-C₁₀)alkyl is reacted with 1 to 1.5 molar equivalents, preferably about 1.1 molar equivalents, of a compound of the Formula R¹⁰Halo (where R¹⁰ is as defined in the Summary of the Invention, and Halo is chloro, bromo, or iodo), preferably R¹⁰Br (for example a 1-cyano-2-halobenzene, preferably 1-cyano-2-bromobenzene), and a transition metal catalyst (such as di-[triphenyl phosphine] palladium chloride, palladium on carbon, or tetrakis[triphenylphosphine] palladium, preferably tetrakis[triphenylphosphine] palladium). The reaction is carried out in an inert solvent (such as tetrahydrofuran, ethanol, and/or toluene; preferably a mixture of toluene and ethanol), in the presence of an excess of a mild base (such as sodium or potassium carbonate, or sodium bicarbonate, preferably sodium carbonate). The reaction is carried out at a temperature of 60°C to reflux, preferably at about reflux for 6 to 48 hours, preferably about 24 hours. When the reaction is substantially complete the indole of Formula (11) where R¹ is (C₁-C₁₀)alkyl, (C₃-C₈)cycloalkyl or (C₃-C₈)cycloalkyl(C₁-C₁₀)alkyl is isolated by conventional means.

### Preparation of Compounds of Formula (12) where R¹ is (C₁-C₁₀)alkyl, (C₃-C₈)cycloalkyl or (C₃-C₈)cycloalkyl(C₁-C₁₀)alkyl and X is -CO₂H

To prepare a compound of Formula (12) where R¹ is (C₁-C₁₀)alkyl, (C₃-C₈)cycloalkyl or (C₃-C₈)cycloalkyl(C₁-C₁₀)alkyl and X is -CO₂H, a compound of Formula (11) where R¹ is (C₁-C₁₀)alkyl, (C₃-C₈)cycloalkyl or (C₃-C₈)cycloalkyl(C₁-C₁₀)alkyl is reacted with 1 to 3 molar equivalents, preferably about 1.5 molar equivalents, of phosgene. The reaction is carried out in an inert solvent (such as ether, methylene chloride, or tetrahydrofuran; preferably tetrahydrofuran) at -20°C to 10°C, preferably about 0°C, for about 8 to 48 hours, preferably overnight. When the reaction is substantially complete the 3-carboxyindole of Formula (12) where R¹ is (C₁-C₁₀)alkyl, (C₃-C₈)cycloalkyl or (C₃-C₈)cycloalkyl(C₁-C₁₀)alkyl and X is -CO₂H is isolated by conventional means.

### Preparation of Compounds of Fomula (12) where R¹ is (C₁-C₁₀)alkyl, (C₃-C₈)cycloalkyl or (C₃-C₈)cycloalkyl(C₁-C₁₀)alkyl and X is -CO₂R⁴

To prepare a compound of Formula (12) where R¹ is (C₁-C₁₀)alkyl, (C₃-C₈)cycloalkyl or (C₃-C₈)cycloalkyl (C₁-C₁₀)alkyl and X is - CO₂R⁴, in which R⁴ is (C₁-C₁₀)alkyl, the solution of a compound of Formula (12) where X is -COCl in dichloromethane, prepared as shown above, is cooled to 0 to 10°C, preferably about 5°C, and reacted with an alcohol of formula R⁴OH, where R⁴ is (C₁-C₁₀)alkyl. When the reaction is substantially complete, the 3-(carbo-loweralkoxy)indole derivative of Formula (12), where R¹ is (C₁-C₁₀)alkyl, (C₃-C₈)cycloalkyl or (C₃-C₈)cycloalkyl (C₁-C₁₀)alkyl and X is -CO₂R⁴, in which R⁴ is (C₁-C₁₀)alkyl, is isolated and purified by conventional means, preferably chromatography.

### Preparation of Compounds of Formula (12) where R¹ is (C₁-C₁₀)alkyl, (C₃-C₈)cycloalkyl or (C₃-C₈)cycloalkyl(C₁-C₁₀)alkyl and X is -CONR⁵R⁶

To prepare a compound of Formula (12) where R¹ is (C₁-C₁₀)alkyl, (C₃-C₈)cycloalkyl or (C₃-C₈)cycloalkyl(C₁-C₁₀)alkyl and X is - CONR⁵R⁶, in which R⁵ and R⁶ are as defined in the Summary of the Invention, the procodure shown above is followed, substituting an amine of formula R⁵R⁶NH for the alcohol of formula R⁴OH.

### Preparation of Compounds of Formula (12) where R¹ is (C₁-C₁₀)alkyl, (C₃-C₈)cycloalkyl or (C₃-C₈)cycloalkyl(C₁-C₁₀)alkyl and X is -COCF₃

To prepare a compound of Formula (12) where R¹ is (C₁-C₁₀)alkyl, (C₃-C₈)cycloalkyl or (C₃-C₈)cycloalkyl (C₁-C₁₀)alkyl and X is - COCF₃, a compound of Formula (11) where R¹ is (C₁-C₁₀)alkyl, (C₃-C₈)cycloalkyl or (C₃-C₈)cycloalkyl(C₁-C₁₀)alkyl is reacted with 1 to 1.5 molar equivalents, preferably about 1.1 molar equivalents, of trifluoroacetic anhydride. The reaction is carried out in an aprotic solvent (such as ether, tetrahydrofuran, or DMF; preferably DMF) at -10°C to 20°C, preferably about 0°C, for about 5 to 30 minutes, preferably about 10 minutes. When the reaction is substantially complete the 3-trifluoroacetylindole of Formula (12) where R¹ is (C₁-C₁₀)alkyl, (C₃-C₈)cycloalkyl or (C₃-C₈)cycloalkyl(C₁-C₁₀)alkyl and X is -COCF₃, is isolated by conventional means.

### Preparation of Compounds of Formula (12) where R¹ is (C₁-C₁₀)alkyl, (C₃-C₈)cycloalkyl or (C₃-C₈)cycloalkyl(C₁-C₁₀)alkyl and X is Halo

To prepare a compound of Formula (12) where R¹ is (C₁-C₁₀)alkyl, (C₃-C₈)cycloalkyl or (C₃-C₈)cycloalkyl(C₁-C₁₀)alkyl and X is halo, a compound of Formula (11) where R¹ is (C₁-C₁₀)alkyl, (C₃-C₈)cycloalkyl or (C₃-C₈)cycloalkyl (C₁-C₁₀)alkyl is reacted with 1 to 1.5 molar equivalents, preferably about 1.1 molar equivalents, of an N-halosuccinimide. The reaction is carried out in an aprotic solvent (such as ether, tetrahydrofuran, or DMF; preferably DMF) at 0°C to 30°C, preferably about 20°C, for about 5 to 30 hours, preferably about 16 hours. When the reaction is substantially complete the 3-halo compound of Formula (12) where R¹ is (C₁-C₁₀)alkyl, (C₃-C₈)cycloalkyl or (C₃-C₈)cycloalkyl(C₁-C₁₀)alkyl and X is halo is isolated by conventional means.

### Alternative Preparation of Compounds of Formula (11)

Alternatively, compounds of Formula (11) may also be prepared as shown below in Reaction Scheme IA. 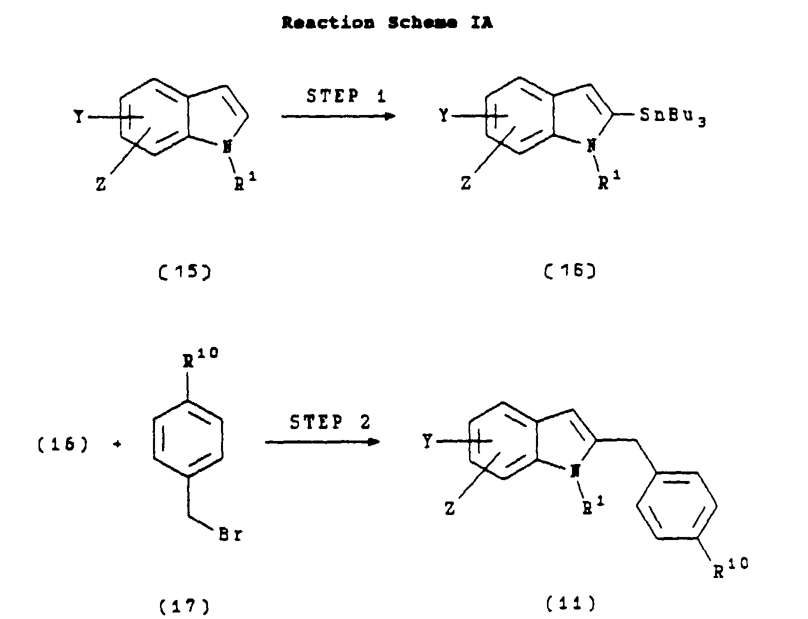 wherein R¹ is (C₁-C₁₀)alkyl, (C₃-C₈)cycloalkyl or (C₃-C₈)cycloalkyl(C₁-C₁₀)alkyl and R¹⁰, Y and Z are as defined above in the Summary of the Invention.

### Starting Materials

The starting compounds of Formula (15) are commercially available from, for example, the Aldrich Chemical Company.

The starting compound of Formula (17) is commercially available from, for example, Lancaster Chemicals, or may be prepared as shown in Example 17, or from compounds of Formula (20) or (28) by means well known in the art, for example by reducing (20) to an alcohol and converting it to a leaving group, for example methanesulfonate, bromo, and the like.

### Preparation of Compounds of Formula (16)

To prepare a compound of Formula (16) where R¹ is (C₁-C₁₀)alkyl, (C₃-C₈)cycloalkyl or (C₃-C₈)cycloalkyl(C₁-C₁₀)alkyl a compound of Formula (15) where R¹ is (C₁-C₁₀)alkyl, (C₃-C₈)cycloalkyl or (C₃-C₈)cycloalkyl(C₁-C₁₀)alkyl is first reacted with about 1 molar equivalent of n-butyl lithium. The reaction is carried out in an aprotic solvent (such as ether, tetrahydrofuran, preferably tetrahydrofuran) mixed with hexamethylphosphoramide (10%) at -10°C to 5°C, preferably about -5°C, for about 30 minutes. The resultant anion is then reacted with 1 to 1.5 molar equivalents, preferably about 1.1 molar equivalents, of tributyl tin chloride, for about 1 hour at about room temperature. When the reaction is substantially complete the compound of Formula (16) where R¹ is (C₁-C₁₀)alkyl, (C₃-C₈)cycloalkyl or (C₃-C₈)cycloalkyl (C₁-C₁₀)alkyl is isolated by conventional means.

### Preparation of Compounds of Formula (11)

To prepare a compound of Formula (11) where R¹ is(C₁-C₁₀)alkyl, (C₃-C₈)cycloalkyl or (C₃-C₈)cycloalkyl (C₁-C₁₀)alkyl, a compound of Formula (16) where R¹ is (C₁-C₁₀)alkyl, (C₃-C₈)cycloalkyl or (C₃-C₈)cycloalkyl (C₁-C₁₀)alkyl is reacted with 1 to 1.5 molar equivalents, preferably about 1.1 molar equivalents, of a compound of the Formula (17) (where R¹⁰ is as defined in the Summary of the Invention), and a transition metal catalyst (such as di-[triphenyl phosphine] palladium chloride, palladium on carbon, or tetrakis[triphenylphosphine] palladium, preferably tetrakis[triphenylphosphine] palladium). The reaction is carried out in an inert solvent (such as tetrahydrofuran, ether, dimethoxyethane; preferably tetrohydrofuran], at a temperature of 50°C to reflux, preferably at about reflux for 30 minutes to 8 hours, preferably about 2 hours. When the reaction is substantially complete the indole of Formula (11) where R¹ is (C₁-C₁₀)alkyl, (C₃-C₈)cycloalkyl or (C₃-C₈)cycloalkyl (C₁-C₁₀)alkyl is isolated by conventional means.

### II. Alternative Preparation of Compounds of Formula (Ia) Compounds of Formula (Ia) where X is -CO₂H may also be prepared from the intermediates of Formula (12a) (a compound of Formula (12) where X is -CO₂H), the preparation of which is shown below in Reaction Scheme IB.

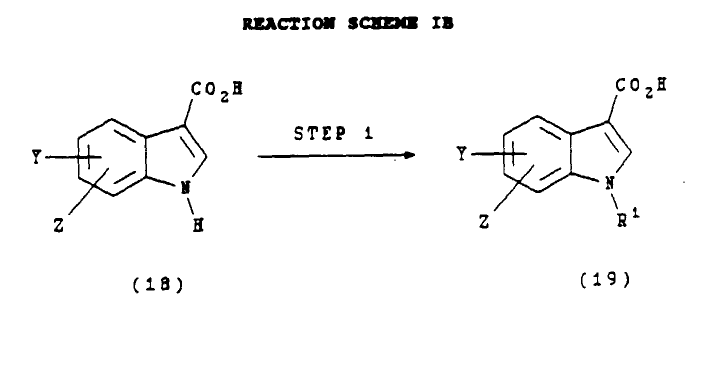

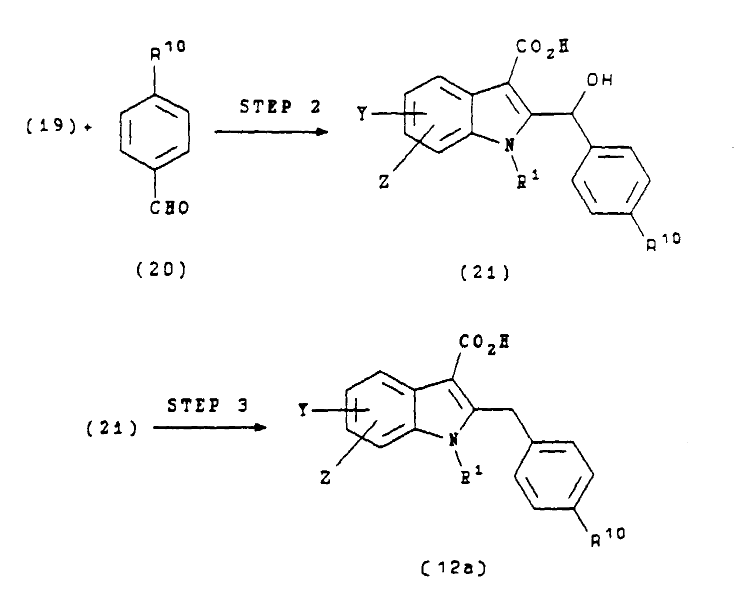

wherein R¹ is (C₁-C₁₀)alkyl, (C₃-C₈)cycloalkyl or (C₃-C₈)cycloalkyl(C₁-C₁₀)alkyl, R¹⁰, Y and Z are as defined above in the Summary of the Invention.

### Starting Materials

The compounds of Formula (18) are commercially available, for example, from Aldrich Chemical Company. Alternatively, they may be prepared conventionally, for example by the methods described in *Synthesis,* Vol. 10, p 871 (1991), or *Organic Preparations and Procedures*, No. 2, Vol. 4, p 297 (1970).

The reactant of Formula (20) where R¹⁰ is 2-cyanophenyl used in Step 2 of Reaction Scheme 1A may be prepared as follows:

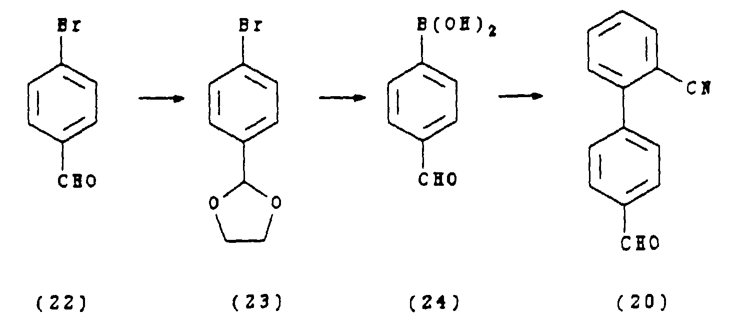

### Preparation of Compounds of Formula (23)

Step 1 of the preparation of the 4-substituted aldehyde of Formula (20) involves reaction of 4-bromobenzaldehyde (22) (which is commercially available, for example, from Aldrich Chemical Company) with ethylene glycol in an inert solvent, preferably toluene, at reflux temperature for about 2 hours in the presence of an acid catalyst, preferably p-toluene sulfonic acid, after which the ethylene ketal of Formula (23) is isolated by conventional means.

### Preparation of Compounds of Formula (24)

A solution of the compound of Formula (23) in a dry ethereal solvent, preferably tetrahydrofuran, is cooled to -60°C, and an equivalent amount of n-butyllithium (preferably in hexane) is added. The mixture is then stirred at -70°C for 30 minutes, after which time a suspension is formed. To the suspension at -70°C is then added tributylborate. The mixture is stirred for additional 3 hours as it gradually warms to 0°C, after which the dihydroxyborane derivative of Formula (24) is isolated by conventional means.

### Preparation of Compounds of Formula (20)

A compound of Formula (20) is then obtained by stirring a mixture of a compound of Formula (24), 2-bromobenzonitrile, tetrakis(triphenylphosphine)palladium(0), and sodium carbonate in an inert solvent, preferably toluene, at reflux temperature for 24 hours. The compound of Formula (20) is then isolated by conventional means.

### Preparation of Compounds of Formula (19)

To prepare a compound of Formula (19), a compound of Formula (18) is first added to a suspension of NaH in an inert solvent preferably DMF in an ice bath. To the thickened suspension, about 1 to 1.5 molar equivalents, preferably about 1.1 molar equivalents, of a (C₁-C₁₀)alkyl, (C₃-C₈)cycloalkyl or (C₃-C₈)cycloalkyl(C₁-C₁₀)alkyl halide of formula R¹Halo, where Halo is chloro, bromo, or iodo, is added at 0°C. The mixture is then stirred at about 0-30°C, preferably at about room temperature for about 6 to 20 hours, preferably about 10 hours. When the reaction is substantially complete, the 3-carboxyindole of Formula (19) where R¹ is (C₁-C₁₀)alkyl, (C₃-C₈)cycloalkyl or (C₃-C₈)cycloalkyl(C₁-C₁₀)alkyl is isolated by conventional means.

### Preparation of Compounds of Formula (21)

To prepare a compound of Formula (21), diisopropylamine in an inert solvent, preferably tetrahydrofuran, is first cooled to -50°C under argon. To this solution n-butyllithium is added and the temperature of the solution is adjusted to -40°C. A compound of Formula (19) is then added to this solution under continuous stirring for about 1 hour, after which a compound of Formula (21) is isolated by conventional means.

### Preparation of Compounds of Formula (12a)

To prepare a compound of Formula (12a), a compound of Formula (21) is reacted with triethylsilane and boron trifluoride etherate in an inert solvent such as methylene chloride. The mixture is stirred for 6 to 10 hours at room temperature. The compound of Formula (12a) thus prepared is isolated by conventional means.

### PREPARATION OF COMPOUNDS OF FORMULA (I)

The preparation of the compounds of Formula (I) from the appropriate intermediates is shown below. It should be understood that the various substituents defined as X may be introduced before conversion of the cyano group to carboxy. However, the various substituents defined as X may be introduced into the ring before or after the conversion of the cyano group to tetrazole. Additionally, compounds where X is -CO₂H can be converted to compounds where X is, for example, -CO₂R⁴, -CONR⁵R⁶, etc, at any stage of the syntheses.

### Preparation of Compounds of Formula (I)

### A. Preparation of (I) where R¹ is (C₁-C₁₀)alkyl, (C₃-C₈)cycloalkyl or (C₃-C₈)cycloalkyl(C₁-C₁₀)alkyl and R² is 2"-(1H-tetrazol-5-yl)biphenyl-4'-ylmethyl

The compounds of Formula (I) where R¹ is (C₁-C₁₀)alkyl, (C₃-C₈)cycloalkyl or (C₃-C₈)cycloalkyl (C₁-C₁₀)alkyl and R² is 2"-(1H-tetrazol-5-yl)biphenyl-4'-ylmethyl identified as Formula (Ia), may be prepared from the intermediates of Formula (11), (12) or (12a) as shown below in Reaction Scheme VI. 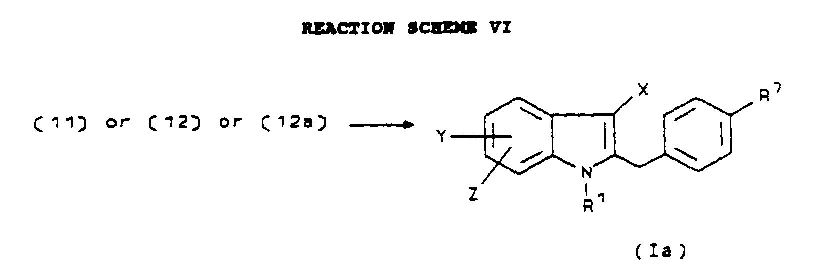 where R⁷ is 2-(1H-tetrazol-5-yl)phenyl; and
R¹, X, Y and Z are as defined in the Summary of the Invention.

### Preparation of Compounds of Formula (Ia) where R⁷ is 2-(1H-tetrazol-5-yl)phenyl

To prepare a compound of Formula (Ia) where R⁷ is 2-(1H-tetrazol-5-yl)phenyl, a compound of Formula (11), (12) or (12a) is reacted with about 1-10 molar equivalents, preferably about 5 molar equivalents, of a trialkyl tin azide, preferably tributyltin azide, in an inert solvent (such as hexane, benzene, or xylene, preferably xylene). The reaction is carried out at a temperature of 80-150°C, preferably at about reflux temperature, for about 8-48 hours, preferably about 20 hours. The mixture is then cooled to about 0°C, and an acid (such as potassium fluoride-tetrafluoroboric acid/diethyl ether complex, sulfuric acid, or hydrochloric acid, preferably hydrochloric acid or potassium fluoride-tetrafluoroboric acid/diethyl ether complex) is added, and stirred for an additional 5-60 minutes, preferably about 15 minutes. When the reaction is substantially complete the indole of Formula (Ia) is isolated and purified by conventional means.

### Preparation of Compounds of Formula (I) where X is (C₁-C₁₀)alkyl

The preparation of compounds of Formula (I) where X is (C₁-C₁₀)alkyl may be accomplished by appropriately modifying the starting materials of the above Reaction Schemes.

### Preparation of Compounds of Formula (I) where X is halo

The preparation of compounds of Formula (I) where X is halo may be accomplished by halogenating an appropriate intermediate in the above Reaction Schemes.

### Isolation and Purification of the Compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the Examples hereinbelow. However, other equivalent separation or isolation procedures could, of course, also be used.

### Salts of Compounds of Formula (I)

The compounds of Formula (I) may be converted to a corresponding base addition salt by virtue of the presence of the acidic tetrazole group, and in some instances because of the presence of a carboxyl group. Such salts may be prepared from either inorganic or organic bases. Salts derived from inorganic bases include, but are not limited to, the sodium, potassium, lithium, ammonium, calcium, magnesium, ferrous, zinc, copper, manganous, aluminum, ferric, and manganic salts, and the like. Salts derived from organic bases include, but are not limited to, salts of primary, secondary and tertiary amines, substituted amines including naturally-occurring substituted amines, and cyclic amines, including isopropylamine, trimethylamine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, N-ethylpiporidine, and the like.

The conversion is preferably accomplished by treatment of the free compound in an inert organic solvent with a stoichiometric amount of an appropriate base in an inert organic solvent (such as methanol or ethanol, preferably methanol) at a temperature of about 0-50°C, preferably ambient temperature. The resulting salt may be brought out of solution with a less polar solvent, by lyophilization of the solution, or by simply evaporating the solvent.

In summary, the compounds of the present invention are made by the procedures outlined below:
1. A process for preparing compounds of the Formula (I), wherein:
   R¹ is (C₁-C₁₀)alkyl, (C₃-C₈)cycloalkyl or (C₃-C₈)cycloalkyl(C₁-C₁₀)alkyl;
   R² is 2"-(1H-tetrazol-5-yl)biphenyl-4'-ylmethyl
   X is hydrogen, (C₁-C₁₀)alkyl, halogen, -C(O)CF₃, -CO₂R⁴, or -C(O)NR⁵R⁶;
   Y is hydrogen, (C₁-C₁₀)alkyl, (C₁-C₁₀)alkoxy, hydroxy, halogen, or -CO₂R⁴;
   Z is hydrogen, (C₁-C₁₀)alkyl, lower alkoxy, or halogen; wherein
      R⁴ is hydrogen or lower alkyl;
      R⁵ is hydrogen, lower alkyl, or -CH₂CO₂R⁴;
      R⁶ is hydrogen or lower alkyl; or
      R⁵ and R⁶ taken together with the nitrogen to which they are attached represent a heterocycle;
      comprises:
      reacting a compound of the formula: 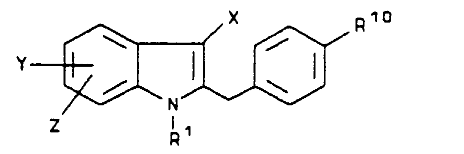 where R¹ is (C₁-C₁₀)alkyl, (C₃-C₈)cycloalkyl or (C₃-C₈)cycloalkyl (C₁-C₁₀)alkyl; and R¹⁰, X, Y and Z are as defined in the Summary of the Invention;
      with a trialkyl tin azide followed by treatment with an acid.
6. A process for preparing compounds of the Formula (I) wherein:
   R¹ is (C₁-C₁₀) alkyl, (C₃-C₈)cycloalkyl or (C₃-C₈)cycloalkyl(C₁-C₁₀)alkyl and
   R² is 2"-(1H-tetrazol-5-yl)biphenyl-4'-ylmethyl
   R⁸ is -CO₂H; and
   R⁴, X, Y and Z are as defined above;
   comprises:
   reacting a compound of the formula: 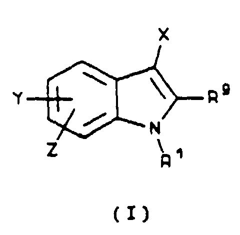 where R⁹ is as defined in the Summary of the Invention;
   with a base.
7. A process for preparing compounds of the Formula (I) wherein:
   R¹ is (C₁-C₁₀) alkyl, (C₃-C₈)cycloalkyl or (C₃-C₈)cycloalkyl(C₁-C₁₀)alkyl and
   R² is 2"-(1H-tetrazol-5-yl)biphenyl-4'-ylmethyl
   X is -CO₂R⁴ or -C(O)NR⁵R⁶; and
   R⁴, Y and Z are as defined above;
   comprises:
   reacting a compound of the formula: 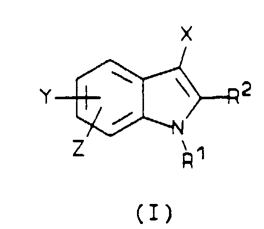 where X is hydrogen;
   with phosgene, optionally followed by an alcohol of formula R⁴OH, where R⁴ is (C₁-C₁₀)alkyl, or an amine of formula R⁵R⁶NH, where R⁵ and R⁶ are as defined in the Summary of the Invention.
8. Alternatively, a process for preparing compounds of the Formula (I) wherein R¹, R², R³, X, Y and Z are as defined in the Summary of the Invention, constitutes:
   (a) reacting the compound of Formula (I) with a base to give a pharmaceutically acceptable base addition salt; or
   (b) reacting a base addition salt of a compound of Formula (I) with an acid to give the corresponding free acid; or
   (c) converting a base addition salt of a compound of Formula (I) to another pharmaceutically acceptable base addition salt of Formula (I).

### Utility and Administration

### A. General Utility

The compounds of the present invention, and the pharmaceutically acceptable salts thereof, exhibit useful pharmacological properties in mammals, including use as cardiovascular agents, such as for treating hypertension, congestive heart failure, and chronic renal disease. In view of the local role of the renin angiotensin system in smooth muscle in the alimentary tract and in the brain, the compounds are also useful for treating disorders of the brain such as cognitive disorders, including mood disorders such as anxiety and depression, as well as disorders of the alimentary tract, including motility and secretory disorders and inflammatory bowel disease. The compounds of the present invention may also be used to treat chronic renal failure, glaucoma, as well as neuroblastoma and other growth disorders.

The ability of the compounds of Formulae (I) to effectively inhibit the binding of angiotensin II to its receptors can be determined by a variety of in *vitro* and in *vivo* assays that are known to those of ordinary skill in the art. In particular, assays which inhibit the response of isolated smooth muscle and cardiac muscle to angiotensin II, and assays which inhibit hypertensive responses to angiotensin II in animal models are predictive of therapeutic utility.

### B. Testing

*In vitro* assays for determining binding of the present compounds at angiotensin II receptors follow a method which is essentially that described by Gunther, *J*. *Biol. Chem.* 259:7622 (1984) and Whitebread et al., *Biochem. Biophys. Res. Comm.* 163:284 (1989). This method employs [¹²⁵I]-Sar¹ IIe⁸ AII in membrane preparations from rat liver (AT-1 receptors) and bovine cerebellum (AT-2 receptors). Binding affinity of a test compound to angiotensin II receptor sites is thus demonstrated.

*In vitro* functional assays for determining antagonist activity of the present compounds at receptors for angiotensin follow a method which is essentially that described by Chiu at al., *J*. *Pharmacol*. *Exp. Ther.* 252:711 (1990) and Kamikawa et al., *Gastroenterology* 88:706 (1985). Isolated ring segments of rabbit thoracic aorta and isolated segments of guinea pig ileum are exposed to angiotensin II with and without test compounds, and a contractile response is evoked. Inhibition of contraction demonstrates angiotensin II receptor antagonist activity of the test compound.

In order to evaluate the *in vivo* anti-hypertensive activity of the present compounds, male normotensive rats are subjected to complete left renal artery ligation essentially as described by Wong et al., *J*. *Pharmacol*, *Exp*. *Ther*. 252:726-732 (1990), and a test compound is administered. Lowering of blood pressure demonstrates *in vivo* hypotensive activity of the test compound. In addition, *in vivo* testing can be conducted on conscious normotensive rats to which angiotensin II and a test compound are administered. *In vivo* activity is demonstrated by inhibition of the pressor response to angiotensin II by the test compound.

Cognition enhancing activity is determined using a modification of the Morris Water Maze spatial learning-memory assay (e.g., see Morris, R.M.G, *J*. *Neurosci. Methods* 1984, Vol. 11, pp 47-60). This procedure tests rats in six trials daily for two consecutive days, recording the time to find a hidden platform. The cognitive enhancement assay is described in Example 20.

### C. General Administration

In applying the compounds of this invention to treatment of the above conditions, any pharmaceutically acceptable mode of administration can be used, either alone or in combination with other pharmaceutically acceptable excipients, including solid, semi-solid liquid or aerosol dosage forms, such as, for example, tablets, capsules, powders, liquids, suspensions, suppositories, aerosols or the like, or in sustained or controlled release dosage forms for the prolonged administration of the compound at a predetermined rate, preferably in unit dosage forms suitable for single administration of precise dosages. The compositions will typically include a conventional pharmaceutical carrier or excipient and an active compound of formula (I) or the pharmaceutically acceptable salts thereof. In addition, these compositions may include other medicinal agents, pharmaceutical agents, carriers, adjuvants, etc., e.g. antihypertensive agents such as angiotensin converting enzyme (ACE) inhibitors, beta-blocking agents and diuretics; bronchodilating agents and antiasthmatics; steroids; antiinflammatories; and non-steroidal antiinflammatories (NSAIDs).

Generally, depending on the intended mode of administration, the pharmaceutically acceptable composition will contain about 0.1% to 90%, preferably about 0.5% to 50%, by weight of a pharmaceutically active compound of this invention, the remainder being suitable pharmaceutical excipients, carriers, etc.

The amount of active compound administered will, of course, be dependent on the subject and disease state being treated, the severity of the affliction, the manner of administration and the judgment of the prescribing physician. However, an effective dosage is in the range of about 0.1 mg to about 100 mg/kg of body weight, preferably about 1 mg to 10 mg/kg. For an average 70 kg human, this would amount to about 7 mg to 7 g per day, or preferably 70-700 mg per day.

The preferred manner of administration for the conditions detailed above is oral, using a convenient daily dosage regimen which can be adjusted according to the degree of affliction. For such oral administration, a pharmaceutically acceptable, non-toxic composition is formed by the incorporation of any of the normally employed excipients, such as, for example, mannitol, lactose, starch, magnesium stearate, sodium saccharine, talcum, cellulose, glucose, gelatin, sucrose, magnesium carbonate, and the like. Such compositions take the form of solutions, suspensions, tablets, pills, capsules, powders, sustained release formulations and the like.

Preferably the compositions will take the form of a pill or tablet and thus the composition will contain, along with the active ingredient, a diluent such as lactose, sucrose, dicalcium phosphate, or the like; a lubricant such as magnesium stearate or the like; and a binder such as starch, gum acacia, polyvinylpyrrolidine, gelatin, cellulose and derivatives thereof, and the like.

Liquid pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, or solubilizing agents, pH buffering agents and the like, for example, sodium acetate, sodium citrate, cyclodextrine derivatives, sorbitan monolaurate, triethanolamine sodium acetate, triethanolamine oleate, etc. Actual methods of preparing such dosage forms are known, or will be apparent, to those skilled in this art; for example, see *Remington's Pharmaceutical Sciences,* Mack Publishing Company, Easton, Pennsylvania, 15th Edition, 1975. The composition or formulation to be administered will, in any event, contain a quantity of the active compound(s) in an amount effective to alleviate the symptoms of the subject being treated.

Dosage forms or compositions containing active ingredient (compounds of formula (I) in the range of 0.005% to 95% with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, starch, magnesium stearate, talcum, cellulose derivatives, glucose, sucrose, magnesium carbonate, and the like. Such compositions take the form of solutions, suspensions, tablets, capsules, powders, sustained release formulations and the like. Such compositions may contain 0.01%-95% active ingredient, preferably 0.1-50%.

For a solid dosage form, the solution or suspension, in for example propylene carbonate, vegetable oils or triglycerides, is preferably encapsulated in a gelatin capsule. Such diester solutions, and the preparation and encapsulation thereof, are disclosed in U.S. Patents Nos. 4,328,245; 4,409,239; and 4,410,545. For a liquid dosage form, the solution, e.g. in a polyethylene glycol, may be diluted with a sufficient quantity of a pharmaceutically acceptable liquid carrier, e.g. water, to be easily measured for administration.

Alternatively, liquid or semi-solid oral formulations may be prepared by dissolving or dispersing compounds of Formula (I) or their salts in vegetable oils, glycols, triglycerides, propylene glycol esters (e.g. propylene carbibate) and the like, and encapsulating these solution or suspension in hard or soft gelatin capsule shells.

Other useful formulations include those set forth in U.S. Patents Nos. Re. 28,819 and 4,358,603.

The formulation can be administered in a single unit dosage form for continuous treatment or in a single unit dosage form ad libitum when relief of symptoms is specifically required.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents, solubility enhancers, and the like, such as for example, sodium acetate, sorbitan monolaurate, triethanolamine oleate, cyclodextrins, etc.

A more recently devised approach for parenteral administration employs the implantation of a slow-release or sustained-release system, such that a constant level of dosage is maintained. See e.g., U.S. Patent No. 3,710,795.

The percentage of active compound contained in such parenteral compositions is highly dependent on the specific nature thereof, as well as the activity of the compound and the needs of the subject. However, percentages of active ingredient of 0.01% to 10% in solution are employable, and will be higher if the composition is a solid which will be subsequently diluted to the above percentages. Preferably the composition will comprise 0.2-2% of the active agent in solution.

Nasal administration is generally characterized by inhalation of the compounds of formula (I) alone or in combination with other pharmaceutically acceptable excipients.

Formulations of compounds of formula (I) may also be administered to the respiratory tract as an aerosol or solution for a nebulizer, or as a microfine powder for insufflation, alone or in combination with an inert carrier such as lactose, for the treatment of reversible airways obstruction and asthma. In such a case, the particles of the formulation have diameters of less than 50 microns, preferably less than 10 microns.

Pharmaceutical compositions suitable for the treatment of glaucoma may be prepared by mixing the active compound with a non-toxic pharmaceutical organic carrier, or with a pharmaceutically acceptable inorganic carrier. Typical of such pharmaceutically acceptable carriers are, for example, water, mixtures of water and water-miscible solvents such as lower alkanols or vegetable oils, glycerol, polyalkylene glycols, hydroxyethyl cellulose, ethyl oleate, carboxymethyl cellulose, polyvinylpyrrolidone, other polymers water miscible such as cellulose derivatives (methylcellulose, carboxymethylcellulose alkaline derivative, hydroxymethylcellulose, hydroxyethylcellulose) and other conventionally employed acceptable carriers. The pharmaceutical composition may also contain non-toxic auxiliary substances such as emulsifying, preserving, wetting, bodying agents and the like, as for example, polyethylene glycols 200, 300, 400, and 600; carbowaxes 1,000, 1,500, 4,000, 6,000 and 10,000; a polyanionic polymer, e.g. a carboxyvinylpolymer having a molecular weight of from about 4,000 to about 6,000,000; antibacterial components such as quaternary ammonium compounds, phenylmercuric salts known to have cold sterilizing properties and which are non-injurious in use; thimerosol, ethyl and propylparaben, benzylic alcohol, phenylethanol; buffering ingredients and isotonic agents such as alkali metal chloride, borate, acetate, gluconate buffers; antioxidant agents such as sodium metabisulfite, butylated hydroxyanisole, butylated hydroxytoluene and other conventional ingredients such as sorbitan monolaurate, triethanolamine oleate, polyoxyethylene sorbitan monopalmitate, dioctyl alkali metal sulfosuccinate, monothioglycerol, ethylenediamine tetraacetic and the like.

### EXAMPLES

The following preparations and examples are given to enable those skilled in the art to more clearly understand and practice the present invention. They should not be considered as a limitation on the scope of the invention, but merely as being illustrative and representative of the preferred embodiments of the present invention.

Unless specified to the contrary, these preparations and examples are carried out under an inert atmosphere, for example nitrogen or argon.

### PREPARATION 1

### Preparation of Compounds of Formula (5)

### A. Preparation of (5) where Y and Z are hydrogen

A solution of o-toluidine (25 g), di-t-butyldicarbonate (56g) in tetrahydrofuran (250 ml) was refluxed for 4 hours. The reaction mixture was poured onto ice water/dilute sodium bicarbonate solution and extracted twice with ether. The organic layer was then washed with brine and dried over magnesium sulfate. The solvent was evaporated under reduced pressure; crystallization of the residue from hexane gave 2-methylphenyl-t-butylcarbamate (42 g), a compound of Formula (5), m.p. 82-83°C.

### B. Preparation of (5), Varying Y and Z

Similarly, following the procedures of Preparation 1A above, but replacing o-toluidine with:
2,3,5-trimethylmethylaniline;
2,4-dimethylmethylaniline;
2,6-dimethylmethylaniline;
4-fluoro-2-methylaniline;
4-chloro-2-methylaniline; and
4-methoxy-2-methylaniline;
the following intermediates of Formula (5) were prepared:
2,3,5-trimethylphenyl-t-butylcarbamate;
2,4-dimethylphenyl-t-butylcarbamate;
2,6-dimethylphenyl-t-butylcarbamate;
2-methyl-4-fluorophenyl-t-butylcarbamate;
2-methyl-4-chlorophenyl-t-butylcarbamate; and
2-methyl-4-methoxyphenyl-t-butylcarbamate.

### C. Preparation of (5), warying Y and Z

Similarly, following the procedures of Preparation 1A above, but replacing o-toluidine with:
3-methoxy-2-methylaniline;
5-hydroxy-2-methylaniline;
6-fluoro-2-methylaniline; and
6-chloro-2,4-dimethylaniline;
the following exemplary intermediates of Formula (5) are prepared:
3-methoxy-2-methylphenyl-t-butylcarbamate;
5-hydroxy-2-methylphenyl-t-butylcarbamate;
6-fluoro-2-methylphenyl-t-butylcarbamate; and
6-chloro-2,4-dimethylphenyl-t-butylcarbamate.

### PREPARATION 2

### Preparation of Compounds of Formula (7)

### A. Preparation of (7) where Y and Z are hydrogen

A solution of 2-methylphenyl-t-butylcarbamate (5.0 g), prepared, for example, as described in Preparation 1, in tetrahydrofuran (100 ml) was cooled to -40°C under argon. s-Butyllithium (37 ml) was added dropwise, maintaining the temperature at -45° to -35°C. After addition of the s-butyllithium, the temperature of the mixture was lowered to -75°C, producing an orange solution.

A solution of N-methyl-N-methoxy-p-bromophenylacetylamide (5.8 g) (Formula (6), prepared, e.g. as described in Reaction Scheme I), in tetrahydrofuran (50 ml) was then added dropwise, maintaining the temperature at below -65°C. After stirring for 10 minutes, the reaction was quenched with water and ether. The mixture was poured onto ice, extracted twice with ether, the organic layer dried over sodium sulphate, and the solvent evaporated to afford an oil. The oil was dissolved in methylene chloride (150 ml) and trifluoroacetic acid (5 ml) was added. The solution was stirred for 30 minutes at room temperature. The mixture was poured onto dilute sodium bicarbonate solution, extracted with ether, the organic layer dried over magnesium sulphate, and the solvent evaporated to produce an oil. The oil was further purified by silica gel chromatography eluting with 15% ethyl acetate in hexane, to give t-butyl 2-(p-bromophenylmethyl)indole-1-carboxylate (6.0 g), a compound of Formula (7), as an oil.

### 8. Preparation of (7), Varying Y and Z

Similarly, following the procedures of Preparation 2A above, but replacing 2-methylphenyl-t-butylcarbamate with other compounds of Formula (5), the following intermediates of Formula (7) were prepared:
t-butyl-(p-bromophenylmethyl)-5,7-dimethylindole-1-carboxylate;
t-butyl-(p-bromophenylmethyl)-5-methylindole-1-carboxylate;
t-butyl-(p-bromophenylmethyl)-7-methylindole-1-carboxylate;
t-butyl-(p-bromophenylmethyl)-5-fluoroindole-1-carboxylate;
t-butyl-(p-bromophenylmethyl)-5-chloroindole-1-carboxylate; and
t-butyl-(p-bromophenylmethyl)-5-methoxyindole-1-carboxylate.

### C. Preparation of (7), warying Y and Z

Similarly, following the procedures of Preparation 2A above, but replacing 2-methylphenyl-t-butylcarbamate with other compounds of Formula (5) the following exemplary intermediates of Formula (7) are prepared:
t-butyl 2-(p-bromophenylmethyl)-4-methoxyindole-1-carboxylate;
t-butyl 2-(p-bromophenylmethyl)-6-hydroxyindole-1-carboxylate;
t-butyl 2-(p-bromophenylmethyl)-7-fluoroindole-1-carboxylate; and
t-butyl 2-(p-bromophenylmethyl)-7-chloro-5-methylindole-1-carboxylate.

### PREPARATION 3

### Preparation of Compounds of Formula (8)

### A. Preparation of (8) where Y and Z are hydrogen

A mixture of t-butyl 2-(p-bromophenylmethyl)indole-1-carboxylate (7) (6.0 g) (prepared, e.g., as described in Preparation 2), 10% sodium hydroxide in water (20 ml), and ethanol (150 ml), was heated under reflux for 6 hours. The mixture was then poured onto a mixture of ice water/dilute brine and extracted twice with ethyl acetate. The extract was washed with brine and dried over magnesium sulphate. The solvent was evaporated under reduced pressure to produce an oil, which then crystallized. The material was recrystallized from methyl t-butyl ether to give 2-(p-bromophenylmethyl)indole (2.2 g), a compound of Formula (8), m.p. 136°C.

### B. Preparation of (8), varying Y and Z

Similarly, following the procedures of Preparation 3A above, but replacing t-butyl 2-(p-bromophenylmethyl)indole-1-carboxylate with other compounds of Formula (7), the following intermediates of Formula (8) were prepared:
2-(p-bromophenylmethyl)-5,7-dimethylindole;
2-(p-bromophenylmethyl)-5-methylindole;
2-(p-bromophenylmethyl)-7-methylindole;
2-(p-bromophenylmethyl)-5-fluoroindole;
2-(p-bromophenylmethyl)-5-chloroindole; and
5-methoxy-2-(p-bromophenylmethyl)indole.

### C. Preparation of (8), varying Y and Z

Similarly, following the procedures of Preparation 3A above, but replacing t-butyl 2-(p-bromophenylmethyl)indole-1-carboxylate with other compounds of Formula (7), the following exemplary intermediates of Formula (8) are prepared:
2-(p-bromophenylmethyl)-4-methoxyindole;
2-(p-bromophenylmethyl)-5-methoxyindole;
2-(p-bromophenylmethyl)-5-methylindole;
2-(p-bromophenylmethyl)-6-hydroxyindole;
2-(p-bromophenylmethyl)-7-fluoroindole; and
2-(p-bromophenylmethyl)-7-chloro-5-methylindole.

### PREPARATION 4

### Preparation of Compounds of Formula (9)

### A. Preparation of (9) where R¹ is n-Butyl and Y and Z are Hydrogen

A mixture of sodium hydride (0.32 g) in DMF (50 ml) was cooled in an ice bath. A solution of 2-(p-bromophenylmethyl)-indole (2.0 g), prepared, e.g., as described in Preparation 3, in DMF, was added, and the mixture stirred for 10 minutes. 1-Iodobutane (0.9 ml) in DMF was added dropwise, and the mixture stirred for 30 minutes. It was then poured into cold dilute HCl and extracted with ether. The extract was washed three times with cold water, dried over magnesium sulphate, and the solvent evaporated to afford an orange-red oil, 1-(n-butyl)-2-(p-bromophenylmethyl)indole (2.5 g), a compound of Formula (9).

### B. Preparation of (9), varying R¹, Y and Z

Similarly, following the procedures of Preparation 4A above, but optionally replacing 2-(p-bromophenylmethyl)indole with other compounds of Formula (8), and optionally replacing iodobutane with other lower alkyl halides, the following intermediates of Formula (9) were prepared:
1-(isopentyl)-2-(p-bromophenylmethyl)indole;
1-ethyl-2-(p-bromophenylmethyl)indole;
1-methyl-2-(p-bromophenylmethyl)indole;
1-(n-pentyl)-2-(p-bromophenylmethyl)indole;
1-(n-butyl)-2-(p-bromophenylmethyl)-5,7-dimethylindole;
1-(n-butyl)-2-(p-bromophenylmethyl)-5-methylindole;
1-(n-butyl)-2-(p-bromophenylmethyl)-7-methylindole:
1-(n-butyl)-2-(p-bromophenylmethyl)-5-fluoroindole;
1-(n-butyl)-2-(p-bromophenylmethyl)-5-chloro-indole;
1-isopropyl-2-(p-bromophenylmethyl)indole;
1-cyclopropylmethyl-2-(p-bromophenylmethyl)indole;
1-cyclopropylmethyl-2-(p-bromophenylmethyl)-5-fluoroindole;
1-cyclopropylmethyl-2-(p-bromophenylmethyl)-5-chloroindole;
1-cyclopropylmethyl-2-(p-bromophenylmethyl)-6-methoxyindole;
1-cyclohexylmethyl-2-(p-bromophenylmethyl)indole;
1-(n-propyl)-2-(p-bromophenylmethyl)indole;
1-(n-octyl)-2-(p-bromophenylmethyl)indole;
1-(n-butyl)-5-methoxy-2-(p-bromophenylmethyl)-3-trifluoroacetylindole; and
1-(n-butyl)-5-methoxy-2-(p-bromophenylmethyl)-indole.

### C. Preparation of (9), varying R¹, Y and Z

Similarly, following the procedures of Preparation 4A above, but optionally replacing 2-(p-bromophenylmethyl)indole with other compounds of Formula (8), and optionally replacing iodobutane with other lower alkyl halides, the following exemplary intermediates of Formula (9) are prepared:
1-ethyl-2-(p-bromophenylmethyl)indole;
1-(2-propyl)-2-(p-bromophenylmethyl)indole;
1-(n-hexyl)-2-(p-bromophenylmethyl)indole;
1-(n-butyl)-2-(p-bromophenylmethyl)-4-methoxyindole;
1-(n-butyl)-2-(p-bromophenylmethyl)-5-methoxyindole;
1-(n-butyl)-2-(p-bromophenylmethyl)-5-methylindole;
1-(n-butyl)-2-(p-bromophenylmethyl)-6-hydroxyindole;
1-(n-butyl)-2-(p-bromophenylmethyl)-7-fluoroindole; and
1-(n-butyl)-2-(p-bromophenylmethyl)-8-chloro-5-methylindole.

### PREPARATION 5

### Preparation of Compounds of Formula (10)

### A. Preparation of (10) where R¹ is n-Butyl and Y and Z are Hydrogen

To a cold (-70°C) stirring solution of 1-(n-butyl)-2-(p-bromophenylmethyl)indole (9) (10.0 g), prepared, e.g., as described in Preparation 4, in tetrahydrofuran (150 ml) was added dropwise n-butyl lithium (18 ml, 2.5 M), and stirring was continued for 15 minutes. Tributyl borate (12.5 ml) in tetrahydrofuran was then added dropwise and the reaction was allowed to warm to 0°C. It was then quenched with 10% HCl (60 ml) (pH 2-3) and stirred for 15 minutes at 20°C. The reaction mixture was extracted three times with ether, the extract washed with water, dried with magnesium sulfate, and the solvent was evaporated to produce an oil, 1-(n-butyl)-2-[4'-(dihydroxyboron)phenylmethyl]indole (7.8 g), a compound of Formula (10).

### B. Preparation of (10), varying R¹, Y and Z

Similarly, following the procedures of Preparation 5A above, but replacing 1-(n-butyl)-2-(p-bromophenylmethyl)indole with other compounds of Formula (9), the following intermediates of Formula (10) were prepared:
1-(isopentyl)-2-[4'-(dihydroxyboron)phenylmethyl]indole;
1-ethyl-2-[4'-(dihydroxyboron)phenylmethyl]indole;
1-methyl-2-[4'-(dihydroxyboron)phenylmethyl]indole;
1-(n-pentyl)-2-[4'-(dihydroxyboron)phenylmethyl]indole;
1-(n-butyl)-2-[4'-(dihydroxyboron)phenylmethyl]-5,7-dimethylindole;
1-(n-butyl)-2-[4'-(dihydroxyboron)phenylmthyl]-5-methylindole;
1-(n-butyl)-2-[4'-(dihydroxyboron)phenylmethyl]-7-methylindole;
1-(n-butyl)-2-[4'-(dihydroxyboron)phenylmethyl]-5-fluoroindole;
1-(n-butyl)-2-[4'-(dihydroxyboron)phenylmethyl]-5-chloro-indole;
1-isopropyl-2-[4'-(dihydroxyboron)phenylmethyl]indole;
1-cyclopropylmethyl-2-[4'-(dihydroxyboron)phenylmethyl]-indole;
1-cyclopropylmethyl-2-[4'-(dihydroxyboron)phenylmethyl]-5-fluoroindole;
1-cyclopropylmethyl-2-[4'-[dihydroxyboron)phenylmethyl]-5-chloroindole;
1-cyclopropylmethyl-2-[4'-(dihydroxyboron)phenylmethyl]-6-methoxyindole;
1-cyclohexylmethyl-2-[4'-(dihydroxyboron)phenylmethyl]-indole;
1-(n-propyl)-2(4'-(dihydroxyboron)phenylmethyl]indole;
1-(n-octyl)-2-[4'-(dihydroxyboron)phenylmethyl]indole;
1-(n-butyl)-5-methoxy-2-[4'-(dihydroxyboron)phenylmethyl]-3-trifluoroacetylindole; and
1-(n-butyl)-5-methoxy-2-[4'-(dihydroxyboron)phenylmethyl]-indole.

### C. Preparation of (10), varying R¹, Y and Z

Similarly, following the procedures of Preparation 5A above, but replacing 1-(n-butyl)-2-(p-bromophenylmethyl)indole with other compounds of Formula (9), the following exemplary intermediates of Formula (10) are prepared:
1-ethyl-2-[4'-(dihydroxyboron)phenylmethyl]indole;
1-(2-propyl)-2-[4'-(dihydroxyboron)phenylmethyl]indole;
1-(n-hexyl)-2-[4'-(dihydroxyboron)phenylmethyl]indole;
1-(n-butyl)-2-[4'-(dihydroxyboron)phenylmethyl]-4-methoxyindole;
1-(n-butyl)-2-[4'-(dihydroxyboron)phenylmethyl]-5-methoxyindole;
1-(n-butyl)-2-[4'-(dihydroxyboron)phenylmethyl]-5-methylindole;
1-(n-butyl)-2-[4'-(dihydroxyboron)phenylmethyl]-6-hydroxyindole;
1-(n-butyl)-2-[4'-(dihydroxyboron)phenylmethyl]-7-fluoroindole; and
1-(n-butyl)-2-[4'-(dihydroxyboron)phenylmethyl]-7-chloro-5-methylindole.

### PREPARATION 6

### Preparation of Compounds of Formula (11)

### A. Preparation of (11) where R¹ is n-Butyl and Y and Z are Hydrogen

1-(n-Butyl)-2-[4'-(dihydroxyboron)phenylmethyl]indole (10), (14.0 g), prepared, e.g., as described in Preparation 5, was dissolved in a mixture of toluene (200 ml), ethanol (40 ml) and 2M sodium carbonate (40 ml). To this mixture was added 1-cyano-2-bromobenzene (5.8 g) and tetrakis[triphenyl phosphine] palladium (0.6 g), and the combined mixture refluxed with vigorous stirring for 24 hours. The reaction mixture was then poured onto water and the layers separated. The organic layer was dried over magnesium sulphate, and the solvent removed under reduced pressure to afford an oil, which was further purified by silica gel chromatography eluting with 10% ethyl acetate in hexane, to yield 1-(n-butyl)-2-(2"-cyanobiphenyl-4'-ylmethyl)indole (8.1 g), a compound of Formula (11), m.p. 64-68°C.

### B. Preparation of (11), varying R¹, Y and Z

Similarly, following the procedures of Preparation 6A above, but replacing 1-(n-butyl)-2-[4'-(dihydroxyboron)phenyl-methyl]indole with other compounds of Formula (10), the following intermediates of Formula (11) were prepared:
1-(isopentyl)-2-(2"-cyanobiphenyl-4'-ylmethyl)indole;
1-ethyl-2-(2"-cyanobiphenyl-4'-ylmethyl)indole;
1-methyl-2-(2"-cyanobiphenyl-4'-ylmethyl)indole;
1-(n-pentyl)-2-(2"-cyanobiphenyl-4'-ylmethyl)indole;
1-(n-butyl)-2-(2"-cyanobiphenyl-4'-ylmethyl)-5,7-dimethylindole;
1-(n-butyl)-2-(2"-cyanobiphenyl-4'-ylmethyl)-5-methylindole;
1-(n-butyl)-2-(2"-cyanobiphenyl-4'-ylmethyl)-7-methylindole;
1-(n-butyl)-2-(2"-cyanobiphenyl-4'-ylmethyl)-5-fluoroindole;
1-(n-butyl)-2-(2"-cyanobiphenyl-4'-ylmethyl)-5-chloro-indole;
1-isopropyl-2-(2"-cyanobiphenyl-4'-ylmethyl)indole;
1-cyclopropylmethyl-2-(2"-cyanobiphenyl-4'-ylmethyl)-indole;
1-cyclopropylmethyl-2-(2"cyanobiphenyl-4'-ylmethyl)-5-fluorindole;
1-cyclopropylmethyl-2-(2"-cyanobiphenyl-4'-ylmethyl)-5-chloroindole;
1-cyclopropylmethyl-2-(2"-cyanobiphenyl-4'-ylmethyl)-6-methoxyindole;
1-cyclohexylmethyl-2-(2"-cyanobiphenyl-4'-ylmethyl)indole;
1-(n-propyl)-2-(2"-cyanobiphenyl-4'-ylmethyl)indole;
1-(n-octyl)-2-(2"-cyanobiphenyl-4'-ylmethyl)indole;
1-(n-butyl)-5-methoxy-2-(2"-cyanobiphenyl-4'-ylmethyl)-3-trifluoroacetylindole; and
1-(n-butyl)-5-methoxy-2-(2"-cyanobiphenyl-4'-ylmethyl)-indole.

### C. Preparation of (11), varying R¹, Y and Z

Similarly, following the procedures of Preparation 6A above, but replacing 1-(n-butyl)-2-[4'-(dihydroxyboron)phenyl-methyl]indole with other compounds of Formula (10), the following exemplary intermediates of Formula (11) are prepared:
1-ethyl-2-(2"-cyanobiphenyl-4' -ylmethyl)indole;
1-(2-propyl)-2-(2"-cyanobiphenyl-4'-ylmethyl)indole;
1-(n-hexyl-2-(2"-cyanobiphenyl-4'-ylmethyl)indole;
1-(n-butyl)-2-(2"-cyanobiphenyl-4'-ylmethyl)-4-methoxyindole;
1-(n-butyl)-2-(2"-cyanobiphenyl-4'-ylmethyl)-5-methoxyindole;
1-(n-butyl)-2-(2"-cyanobiphenyl-4'-ylmethyl)-5-methylindole;
1-(n-butyl)-2-(2"-cyanobiphenyl-4'-ylmethyl)-6-hydroxyindole;
1-(n-butyl)-2-(2"-cyanobiphenyl-4'-ylmethyl)-7-fluoroindole; and
1-(n-butyl]-2-(2"-cyanobiphenyl-4'-ylmethyl)-7-chloro-5-methylindole.

### PREPARATION 7

### Preparation of Compounds of Formula (12)

### A. Preparation of (12) where R¹ is n-Butyl, X is -CO₂H, and Y and Z are Hydrogen

To a solution of 1-(n-butyl)-2-(2"-cyanobiphenyl-4'-ylmethyl)indole (11) (5.0 g), prepared e.g., as described in Preparation 6, in tetrahydrofuran (100 ml) at 0°C was added phosgene solution (10 ml, 20% in toluene) dropwise. The reaction was kept at 0°C overnight. Water (20 ml) was then added and the mixture stirred for 20 minutes. The layers were separated, the aqueous phase extracted with ether, then the combined organic layers washed with dilute sodium hydroxide. The aqueous layer was acidified at 0°C with dilute HCl, and the resultant precipitate was filtered off and washed with water and air dried, to yield 1-(n-butyl)-2-(2"-cyanobiphenyl-4'-ylmethyl)indole-3carboxylic acid (2.4 g), a compound of Formula (12), m.p. 186-188°C.

### B. Preparation of (12) where R¹ is n-Butyl, X is -COCF₃, and Y and Z are Hydrogen

To a solution of 1-(n-butyl)-2-(2"-cyanobiphenyl-4'-ylmethyl)indole (11) (0.9 g), prepared e.g., as described in Preparation 6, in dimethylformamide (20 ml) at 0°C was added trifluoroacetic anhydride (0.4 ml) dropwise. The reaction was stirred for 10 minutes, and poured into ice/water. The precipitate was filtered off, washed with water, and recrystallized from ether/hexane, to give 1-(n-butyl)-2-(2"-cyanobiphenyl-4'-ylmethyl)-3-trifluoroacetylindole (0.85 g), a compound of Formula (12).

### C. Preparation of (12), varying R¹, Y and Z

Similarly, following the procedures of Preparation 7A or 7B above, but replacing 1-(n-butyl)-2-(2"-cyanobiphenyl-4'-ylmethyl)indole with other compounds of Formula (11), the following intermediates of Formula (12) were prepared:
1-(isopentyl)-2-(2"-cyanobiphenyl-4'-ylmethyl)indole-3-carboxylic acid;
1-ethyl-2-(2"-cyanobiphenyl-4'-ylmethyl)indole-3-carboxylic acid;
1-methyl-2-(2"-cyanobiphenyl-4'-ylmethyl)indole-3-carboxylic acid;
1-(n-pentyl)-2-(2"-cyanobiphenyl-4'-ylmethyl)indole-3-carboxylic acid;
1-(n-butyl)-2-(2"-cyanobiphenyl-4'-ylmethyl)-5,7-dimethylindole-3-carboxylic acid;
1-(n-butyl)-2-(2"-cyanobiphenyl-4'-ylmethyl)-5-methylindole-3-carboxylic acid;
1-(n-butyl)-2-(2"-cyanobiphenyl-4'-ylmethyl)-7-methylindole-3-carboxylic acid;
1-(n-butyl)-2-(2"-cyanobiphenyl-4'-ylmethyl)-5-fluoroindole-3-carboxylic acid;
1-(n-butyl)-2-(2"-cyanobiphenyl-4'-ylmethyl)-5-chloro-indole-3-carboxylic acid;
1-isopropyl-2-(2"-cyanobiphenyl-4'-ylmethyl)-indole-3-carboxylic acid;
1-cyclopropylmethyl-2-(2"-cyanobiphenyl-4'-ylmethyl)-indole-3-carboxylic acid;
1-cyclopropylmethyl-2-(2"-cyanobiphenyl-4'-ylmethyl)-5-fluoroindole-3-carboxylic acid;
1-cyclopropylmethyl-2-(2"-cyanobiphenyl-4'-ylmethyl)-5-chloroindole-3-carboxylic acid;
1-cyclopropylmethyl-2-(2"-cyanobiphenyl-4'-ylmethyl)-6-methoxyindole-3-carboxylic acid;
1-cyclohexylmethyl-2-(2"-cyanobiphenyl-4'-ylmethyl)indole-3-carboxylic acid;
1-(n-propyl)-2-(2"-cyanobiphenyl-4'-ylmethyl)-indole-3-carboxylic acid;
1-(n-octyl)-2-(2"-cyanobiphenyl-4'-ylmethyl)-indole-3-carboxylic acid; and
1-(n-butyl)-5-methoxy-2-(2"-cyanobiphenyl-4'-ylmethyl)-indole-3-carboxylic acid.

### D. Preparation of (12), varying R¹, Y and Z

Similarly, following the procedures of Preparation 7A or 7B above, but replacing 1-(n-butyl)-2-(2"-cyanobiphenyl-4'-ylmethyl)indole with other compounds of Formula (11), the following exemplary intermediates of Formula (12) are prepared:
1-ethyl-2-(2"-cyanobiphenyl-4'-ylmethyl)indole-3-carboxylic acid;
1-(2-propyl)-2-(2"-cyanobiphenyl-4'-ylmethyl)indole-3-carboxylic acid;
1-(n-hexyl-2-(2"-cyanobiphenyl-4'-ylmethyl)indole-3-carboxylic acid;
1-(n-butyl)-2-(2"-cyanobiphenyl-4'-ylmethyl)-4-methoxyindole-3-carboxylic acid;
1-(n-butyl)-2-(2"-cyanobiphenyl-4'-ylmethyl)-5-methoxyindole-3-carboxylic acid;
1-(n-butyl)-2-(2"-cyanobiphenyl-4'-ylmethyl)-5-methylindole-3-carboxylic acid;
1-(n-butyl)-2-(2"-cyanobiphenyl-4'-ylmethyl)-6-hydroxyindole-3-carboxylic acid;
1-(n-butyl)-2-(2"-cyanobiphenyl-4'-ylmethyl)-7-fluoroindole-3-carboxylic acid;
1-(n-butyl)-2-(2"-cyanobiphenyl-4'-ylmethyl)-3-trifluoroacetyl-7-fluoroindole; and
1-(n-butyl)-2-(2"-cyanobiphenyl-4'-ylmethyl)-7-chloro-5-methylindole-3-carboxylic acid.

### PREPARATION 8

### Preparation of Compounds of Formula (19)

### A. Preparation of (19) where R¹ is n-Butyl, X is -CO₂H, and Y and Z are Hydrogen

To a suspension of 34.1 g NaH (60% dispersion) in 300ml DMF in an ice/methanol bath was added dropwise over 45 minutes a solution of 55 g indole-3-carboxylic acid in 250 ml DMF. The mixture was stirred for 30 minutes after completion of the addition, after which a further 100 ml of DMF was added.

To the grey thick suspension thus produced was added 40.8 ml of 1-iodobutane at 0°C, and the mixture stirred at room temperature overnight. The suspension than was poured into 2 liter of ice water and acidified with 1 N HCl, extracted with ethyl acetate three times, washed twice with water, and dried (MgSO₄). The solid material was stirred with ethyl ether and filtered, giving 1-(n-butyl)indole-3-carboxylic acid, 65.4 g.

### B. Preparation of (19) varying R¹, Y, and Z

Similarly, following the procedures of Preparation 8A above, but replacing 1-iodobutane with methyl iodide, the following intermediale of Formula (19) was prepared:
1-methylindole-3-carboxylic acid.

### PREPARATION 9

### Preparation of Compound of Formula (20)

### A. Preparation of (20) where R¹⁰ is 2-Cyanophenyl

A mixture of 100 g 4-bromobenzaldehyde, 40 ml ethylene glycol, 800 ml of toluene and a catalytic amount of p-toluenesulfonic acid was refluxed for 2 hours, removing water with a Dean-Stark trap. The mixture was then cooled and poured into cold dilute potassium carbonate solution, extracted three times with ether and dried (MgSo₄). Solvent was removed under reduced pressure, giving 118 g of 4-bromobenzaldehyde ethylene ketal (23) as a solid.

The ketal (23) was dissolved in 200 ml of dry tetrahydrofuran. To this solution was added a solution of 260 ml of 2.5M of n-butyllithium (in hexane) in 1 liter of dry tetrahydrofuran under argon, and stirred at -70°C for 30 minutes. To the suspension thus formed tributylborate (184 ml) was added dropwise over 15 minutes. The mixture was stirred for 3 hours as it gradually warmed to 0°C, and kept at 0°C in an ice bath. The mixture was poured into 10% HCl, stirred for 1 hour, extracted with diethylether three times, and washed with cold 1M NaOH. The NaOH extracts were cooled in ice and acidified to pH 2 with dilute HCl. The product was washed with water and dried, giving 4-(dihydroxyboron)benzaldehyde, (72g), a compound of Formula (24).

The boron compound (24) (65.6 g) and 2-bromobenzonitrile (84 g) were placed into a 3 liter 3-neck flask fitted with a mechanical stirrer containing 20 g of tetrakis(triphenylphosphine)palladium(0), 1 liter of toluene, 480 ml of 2M sodium carbonate solution and 240 ml ethanol. The mixture was gently refluxed under argon with rapid stirring for 24 hours. A further 3g of triphenylphosphine was added and the mixture was continued to reflux overnight. White it was still warm 200 ml of water and 300 ml of tetrahydrofuran was added to the mixture. The organic layer was separated while warm, and the aqueous layer was extracted with ethyl acetate. The organic extracts were combined and dried (MgSO₄). The solid material was recrystallized from a mixture of ethyl acetate and methylene chloride, to yield 55 g of 2-formyl-2'-cyanobiphenyl (99+% pure).

### PREPARATION 10

### Preparation of Compounds of Formula (21)

### A. Preparation of (21) where R¹ is n-Butyl, R¹⁰ is 2-Cyanophenyl, and Y and Z are Hydrogen

To a 3 liter 3-neck flask was added 1 liter of dry tetrahydrofuran and 48.4 ml diisopropylamine. The mixture was cooled to -50°C under argon, and 216 ml of 1.6M of n-butyllithium (in hexane) was added dropwise over 5 minutes. The mixture was then allowed to warm to -10°C, and then recooled to -40°C. A solution of 30 g of 1-butylindole-3-carboxylic acid in 100 ml dry tetrahydrofuran was added dropwise over 5 minutes. The solution was stirred at -40° to -30°C for 1 hour, and then recooled to -70°C. At once was added 28.61 g of 2-formyl-2'-cyanobiphenyl, and the mixture was stirred at -70°C under argon for 2 hours. The mixture then was warmed to -20°C and 500 ml of water was added while stirring. Ethyl acetate was then added to the mixture and the aqueous layer separated, which was extracted three times with ethyl acetate. The ethyl acetate extracts were combined, washed with 1 N HCl, and dried (MgSO₄). The solvent was removed under reduced pressure, and the resulting material recrystallized from a mixture of ethyl acetate and hexane, to yield 36.4 g of 1-(n-butyl)-2-[1-hydroxy-1-(2"-cyanobiphenyl-4'-yl)methyl]indole-3-carboxylic acid.

### PREPARATION 11

### Preparation of Compounds of Formula (11a) and (12a)

### A. Preparation of (12a) where R¹ is n-Butyl, R¹⁰ is 2-Cyanophenyl, and Y and Z are Hydrogen

To a solution of 36.4 g of 1-(n-butyl)-2-[1-hydroxy-1-(2"-cyanobiphenyl-4'-yl)methyl]indole-3-carboxylic acid, obtained for example as in Preparation 10, 150 ml of triethylsilane and 1.5 liters of methylene chloride at 0°C was added dropwise 284 ml of BF₃, etherate over 20 minutes. The mixture was then warmed to room temperature, stirred for 7 hours, and poured slowly into 1 liter of saturated aqueous bicarbonate solution. The basic mixture was acidified with concentrated HCl until pH 2 was attained. The methylene chloride layer was separated, and the remaining material was extracted two times with methylene chloride. The methylene chloride extracts were combined and dried (MgSO₄). The solid material was recrystallized from a mixture of methylene chloride and hexane to yield 22.9 g of 1-(n-butyl)-2-[2"-cyanobiphenyl-4 '-ylmethyl]indole-3-carboxylic acid.

### C. Preparation of (11a) varying R¹, R¹⁰, Y, and Z

Similarly, following the procedures of Preparation 11A above, but replacing 1-(n-butyl)-2-[1-hydroxy-1-(2"-cyanobiphenyl-4'-yl)methyl]indole-3-carboxylic acid with other compounds of Formula (50), the following intermediates of Formula (11a) are prepared:
1-methyl-2-[2"-cyanobiphenyl-4'-ylmethyl]-7-azaindole-3-carboxylic acid;
1-cyclopropylmethyl-2-[2"-cyanobiphenyl-4'-ylmethyl]-7-azaindole-3-carboxylic acid;
1-cyclohexylmethyl-2-[2"-cyanobiphenyl-4'-ylmethyl]-7-azaindole-3-carboxylic acid;
1-(n-butyl)-2-[2"-cyanobiphenyl-4'-ylmethyl]-7-azaindole-3-carboxylic acid.

### PREPARATION 16

### Preparation of Compounds of Formula (16)

### A. Preparation of (16) where R¹ is n-Butyl

A solution of n-butyl lithium in hexane (1.6M, 12.9 ml) was added dropwise to a cooled (-5°C) solution of 3.25 g of 1-n-butylindole in a mixture of THF and hexamethylphosphoramide (9:1), keeping the reaction temperature below 5°C. After the addition was completed, the reaction mixture was stirred for half a hour at -5°C and then 5.85 g of tributyl tin chloride in THF was added dropwise at -5°C. The reaction mixture was allowed to warm to room temperature and stirred for 0.45 hour, quenched with water and extracted with hexane. The organic layer was then washed with brine dried and concentrated, yielding 8.4 g of 1-n-butyl-2-(tributyltin)indole, a compound of Formula (16), as an oil.

### B. Preparation of (16), varying R¹, Y and Z

Similarly, following the procedures of Preparation 16A above, but replacing 1-n-butylindole with other compounds of Formula (15), the following intermediates of Formula (16) are prepared:
1-methyl-2-(tributyltin)indole;
1-isopropyl-2-(tributyltin)indole;
1-cyclopropylmethyl-2-(tributyltin)indole;
1-cyclohexylmethyl-2-(tributyltin)indole; and
1-octyl-2-(tributyltin)indole.

### PREPARATION 17

### Preparation of Compounds of Formula (17)

### A. Preparation of (17) where R¹⁰ is 3-Cyanothiophen-2-yl

A mixture of 2-(4-methylphenyl)-3-cyanothiophene (1.5 g, 7.5 mmol), N-bromosuccinimide (1.34 g, 7.5 mmol), and azabicyclononane (20 mg) in carbon tetrachloride was irradiated with UV light for one hour. The reaction mixture was cooled, and the solid filtered off. The filtrate was concentrated, and the residue purified by flash chromatography, eluting with 5% ethyl acetate/hexane, to yield 2-(4-bromomethylphenyl)-3-cyanothiophene 1.4 g, m.p. 68-75 (slightly impure, used as such).

### B. Preparation of (17), where R¹⁰ is 2-cyanophenyl

Similarly, following the procedures of Preparation 17A above, but replacing 2-(4-methylphenyl)-3-cyanothiophene with other similar compounds, the following intermediate of Formula (17) was prepared:
4'-(bromomethyl)-2"-cyanobiphenyl.

### EXAMPLE 1

### Preparation of Compounds of Formula (Ia)

### A. Preparation of (Ia) where R¹ is n-Butyl, R⁷ is 2-(1H-tetrazol-5-yl)phenyl, X is -CO₂H, and Y and Z are Hydrogen

A mixture of 1-(n-butyl)-2-(2"-cyanobiphenyl-4'-ylmethyl]indole-3-carboxylic acid (12) (0.35 g), xylene (20 ml), and tributyltin azide (2 ml) was heated under reflux for about 20 hours. The mixture was then cooled in an ice bath, HCl in ether was added, and the mixture was stirred for 15 minutes. The mixture was filtered and washed with ether to yield 1-(n-butyl)-2-[2"-(1H-tetrazol-5-yl)biphenyl-4'-ylmethyl]indole-3-carboxylic acid (0.34 g), a compound of Formula (Ia), m.p. 200-203°C.

### C. Preparation of (Ia) where R¹ is n-Butyl, Z is Hydrogen, varying R⁷, X and Y

Similarly, following the procedures of Example 1A or IB above, but replacing 1-(n-butyl)-2-(2"-cyanobiphenyl-4'-ylmethyl)indole-3-carboxylic acid with other compounds of Formula (11), (12), or (12a), the following compounds of Formula (Ia) were prepared:
1-(n-butyl)-2-[2"-(1H-tetrazol-5-yl)biphenyl-4'-ylmethyl]-3-trifluoroacetylindole, m.p. 223-225°C;
1-(n-butyl)-2-[2"-(1H-tetrazol-5-yl)biphenyl-4'-ylmethyl]-indole-3-carbonylaminoacetic acid, m.p. 234-237°C;
1-(n-butyl)-2-[2"-(1H-tetrazol-5-yl)biphenyl-4'-ylmethyl]-indole-3-carbonylaminoacetic acid methyl ester, m.p. 140-144°C;
1-(n-butyl)-2-[2"-(1H-tetrazol-5-yl)biphenyl-4'-ylmethyl]-4-methoxyindole-3-carboxylic acid, m.p. 216-217°C;
1-(n-butyl)-2-[2"-(1H-tetrazol-5-yl)biphenyl-4'-ylmethyl]-6-methoxyindole-3-carboxylic acid, m.p. 221-222°C;
1-(isopentyl)-2-[2"-(1H-tetrazol-5-yl)biphenyl-4'-ylmethyl]indole-3-carboxylic acid, m.p. 210-211°C;
1-ethyl-2-[2"-(1H-tetrazol-5-yl)biphenyl-4'-ylmethyl]indole-3-carboxylic acid, m.p., 234-237°C;
1-methyl-2-[2"-(1H-tetrazol-5-yl)biphenyl-4'-ylmethyl]indole-3-carboxylic acid, m.p., 231-232°C;
1-(n-pentyl)-2-[2"-(1H-tetrazol-5-yl)biphenyl-4'-ylmethyl]indole-3-carboxylic acid, m.p., 209-210°C;
1-(n-butyl)-2-[2"-(1H-tetrazol-5-yl)biphenyl-4'-ylmethyl]-5,7-dimethylindole-3-carboxylic acid, m.p. p 207-209°C;
1-(n-butyl)-2-[2"-(1H-tetrazol-5-yl)biphenyl-4'-ylmethyl]-5-methylindole-3-carboxylic acid, m.p. 207-209°C;
1-(n-butyl)-2-[2"-(1H-tetrazol-5-yl)biphenyl-4'-ylmethyl]-7-methylindole-3-carboxylic acid, m.p., 218-219°C;
1-(n-butyl)-2-[2"-(1H-tetrazol-5-yl)biphenyl-4'-ylmethyl]-5-fluoroindole-3-carboxylic acid, m.p., 216-219°C;
1-(n-butyl)-2-[2"-(1H-tetrazol-5-yl)biphenyl-4'-ylmethyl]-5-chloroindole-3-carboxylic acid, m.p., 228°C;
1-isopropyl-2-[2"-(1H-tetrazol-5-yl]biphenyl-4'-ylmethyl]-indole-3-carboxylic acid, m.p. 174-175°C;
1-cyclopropylmethyl-2-[2"-(1H-tetrazol-5-yl)biphenyl-4'-ylmethyl]indole-3-carboxylic acid, m.p. 273-274°C;
1-cyclopropylmethyl-2-[2"-(1H-tetrazol-5-yl)biphenyl-4'-ylmethyl]-5-fluoroindole-3-carboxylic acid, m.p. 284-285°C;
1-cyclopropylmethyl-2-[2"-(1H-tetrazol-5-yl)biphenyl-4'-ylmethyl]-5-chloroindole-3-carboxylic acid, m.p. 275-276°C;
1-cyclopropylmethyl-2-[2"-(1H-tetrazol-5-yl)biphenyl-4'-ylmethyl]-6-methoxyindole-3-carboxylic acid, m.p. 252-253°C;
1-cyclohexylmethyl-2-[2"-(1H-tetrazol-5-yl)biphenyl-4'-ylmethyl]indole-3-carboxylic acid, m.p. 192-193°C;
1-cyclohexylmethyl-2-[2"-(1H-tetrazol-5-yl)biphenyl-4'-ylmethyl]-5-fluoroindole-3-carboxylic acid, m.p. 163-165°C;
1-cyclohexylmethyl-2-[2"-(1H-tetrazol-5-yl)biphenyl-4'-ylmethyl]-5-chloroindole-3-carboxylic acid, m.p. 137-142°C;
1-(n-propyl)-2-[2"-(1H-tetrazol-5-yl)biphenyl-4'-ylmethyl]indole-3-carboxylic acid, m.p., 233-235°C;
1-(n-octyl)-2-[2"-(1H-tetrazol-5-yl)biphenyl-4'-ylmethyl]-indole-3-carboxylic acid, m.p., 189-190°C.
1-(n-butyl)-5-methoxy-2-[2"-(1H-tetrazol-5-yl)biphenyl-4'-ylmethyl]-3-trifluoroacetylindole, m.p. 214-218°C;
1-(n-butyl)-5-methoxy-2-[2"-(1H-tetrazol-5-yl)biphenyl-4'-ylmethyl)indole-3-carboxylic acid, m.p. 227°C;

### D. Preparation of (Ia), varying R¹, X, Y and Z

Similarly, following the procedures of Example 1A above, but replacing 1-(n-butyl)-2-(2"-cyanobiphenyl-4'-ylmethyl)-indole-3-carboxylic acid with other compounds of Formula (11) or (12), the following exemplary compounds of Formula (Ia) are prepared:
1-ethyl-2-[2"-(1H-tetrazol-5-yl]biphenyl-4'-ylmethyl]indole-3-carboxylic acid;
1-(2-propyl)-2-[2"-(1H-tetrazol-5-yl)biphenyl-4'-ylmethyl]indole-3-carboxylic acid;
1-(n-hexyl)-2-[2"-(1H-tetrazol-5-yl]biphenyl-4'-ylmethyl]indole-3-carboxylic acid;
1-(n-butyl)-2-[2"-(1H-tetrazol-5-yl)biphenyl-4'-ylmethyl]-5-methylindole-3-carboxylic acid;
1-(n-butyl)-2-[2"-(1H-tetrazol-5-yl)biphenyl-4'-ylmethyl]-6-hydroxyindole-3-carboxylic acid;
1-(n-butyl)-2-[2"-(1H-tetrazol-5-yl)biphenyl-4'-ylmethyl]-7-fluoroindole-3-carboxylic acid;
1-(n-butyl)-2-[2"-(1H-tetrazol-5-yl)biphenyl-4'-ylmethyl]-3-trifluoroacetyl-7-fluoroindole;
1-(n-butyl)-2-[2"-(1H-tetrazol-5-yl)biphenyl-4'-ylmethyl]-7-chloro-5-methylindole-3-carboxylic acid;
1-(n-butyl)-3-chloro-2-[2"-(1H-tetrazol-5-yl)biphenyl-4'-ylmethyl]indole-3-carboxylic acid; and
1-(n-butyl)-3-methyl-2-[2"-(1H-tetrazol-5-yl)biphenyl-4'-ylmethyl]indole-3-carboxylic acid.

### EXAMPLE 2

### Preparation of Compounds of Formula (Ia) where X is -CONR⁵R⁶

### A. Preparation of (Ia) where R¹ is n-Butyl, R⁷ is 2-(1H-tetrazol-5-yl)phenyl, X is 3-(N-piperidinocarbonyl), and Y and Z are Hydrogen

A solution of 1-(n-butyl)-2-[2"-(1H-tetrazol-5-yl)biphenyl-4'-ylmethyl]indole-3-carboxylic acid (0.4 g) (prepared, e.g, as described in Example 1), and carbonyldiimidazole (0.2 g) in acetonitrile (10 ml) was heated under reflux for 1 hour Piperidine (0.14 ml) and triethylamine (0.2 ml) were added, and the mixture was heated under reflux overnight. The mixture was then poured onto cold dilute HCl and extracted twice with ethyl acetate. The extract was washed with sodium bicarbonate, dried over magnesium sulfate, and evaporated under reduced pressure to yield 1-(n-butyl)-3-(N-piperidinocarbonyl)-2-[2"-(1H-tetrazol-5-yl)biphenyl-4'-ylmethyl]indole (0.43 g), a compound of Formula (Ia), m.p. 127-130°C.

### B. Preparation of (Ia) where R¹ is n-Butyl, R⁷ is 2-(1H-tetrazol-5-yl)phenyl, X is 3-(N-methylaminocarbonyl), and Y and Z are Hydrogen

Similarly, following the procedures of Example 2A above, but replacing piperidine with methylamine, the following compound of Formula (Ia) was prepared:
1-(n-butyl)-3-(N-methylaminocarbonyl)-2-[2"-(1H-tetrazol-5-yl)biphenyl-4'-ylmethyl]indole, m.p. 166°C.

### C. Preparation of (Ia), varying R¹, X, Y and Z

Similarly, following the procedures of Example 2A above, but optionally replacing 1-(n-butyl)-2-[2"-(1H-tetrazol-5-yl)biphenyl-4'-ylmethyl]indole-3-carboxylic acid with other compounds of Formula (Ia) where X is -CO₂H, and optionally replacing piperidine with alkylamines of the formula R⁵R⁶NH, the following exemplary compounds of Formula (Ia) are prepared:
n-propyl 1-methyl-2-[2"-(1H-tetrazol-5-yl)biphenyl-4'-ylmethyl]indole-3-carboxylate;
ethyl 1-(n-butyl]-2-[2"-(1H-tetrazol-5-yl)biphenyl-4'-ylmethyl]indole-3-carboxylate;
1-ethyl-3-(N-methylcarboxamido)-2-[2"-(1H-tetrazol-5-yl)biphenyl-4'-ylmethyl]indole-3-carboxylic acid;
methyl 1-(2-propyl)-2-[2"-(1H-tetrazol-5-yl)biphenyl-4'-ylmethyl]indole-3-carboxylate; and
1-(n-hexyl-3-(N-hexylcarboxamido)-2-[2"-(1H-tetrazol-5-yl)biphenyl-4'-ylmethyl]indole-3-carboxylic acid.

### EXAMPLE 11

### PREPARATION OF SALTS OF COMPOUNDS OF FORMULA (I)

### A. Conversion of 1-(n-butyl)-6-methoxy-2-[2"-(1H-tetrazol-5-yl)biphenyl-4'- ylmethyl]-indole to its Potassium Salt

1-(n-Butyl)-6-methoxy-2-[2"-(1H-tetrazol-5-yl)biphenyl-4'-ylmethyl]-indole (100 mg), prepared, e.g, as described in Example 1, was dissolved in methanol (20 ml), and a solution of potassium hydroxide (4.65 ml, 0.1 M) in methanol was added. The solvent was evaporated to afford an oil, which was triturated with ethyl acetate to yield a white solid, potassium 1-(n-butyl)-6-methoxy-2-[2"-(1H-tetrazol-5-yl]biphenyl-4'-ylmethyl]-indole as a foam.

### B. In a similar manner, all compounds of Formula

(I) may be converted to their base addition salts by treatment with an appropriate base, for example, sodium bicarbonate, potassium carbonate, lithium hydroxide, ammonium hydroxide, calcium hydroxide, magnesium hydroxide, and the like. Base addition salts derived from organic bases include those formed from primary, secondary and tertiary amines, such as isopropylamine, diethylamine, trimethylamine, pyridine, cyclohexylamine. ethylene diamine, monoethanolamine, diethanolamine, triethanolamine, and the like.

In Examples 12 through 16 the active ingredient is 1-(n-butyl)-2-[2"-(1H-tetrazol-5-yl)biphenyl-4'-ylmethyl]-indole-3-carboxylic acid. Other compounds of Formula (I) and the pharmaceutically acceptable salts thereof, may be substituted therein.

### EXAMPLE 12

| **Composition for Oral Administration** | |
|---|---|
| The composition contains: | % wt./wt. |
| Active ingredient | 40% |
| Lactose | 59.5% |
| Magnesium stearate | 0.5% |

The two ingredients are milled, mixed and dispensed into capsules containing 125-250 mg each; one capsule would approximate a total daily dosage of 50-100 mg.

### EXAMPLE 13

| **Composition for Oral Administration** | |
|---|---|
| The composition contains: | % wt./wt. |
| Active ingredient | 50.0% |
| Microcrystalline cellulose | 45.0% |
| Croscarmellose sodium | 2.0% |
| PVP (polyvinylpyrrolidine) | 2.0% |
| Magnesium stearate | 1.0% |

The first four ingredients above are combined and granulated using water as solvent. The formulation is then dried, blended with the magnesium stearate, and formed into tablets (containing 50-100 mg of active compound) with an appropriate tableting machine.

The compounds of the present invention may also be formulated is a solution for oral administration as follows:

| | |
|---|---|
| Active Ingredient | 10 mg/ml |
| Sucrose | 10 mg/ml |
| Sorbitol | 100 mg/ml |
| Sodium benzoate | 10 mg/ml |

HCl or NaOH to adjust pH to 3 - 7; q.s. to 1 ml with H₂O.

### EXAMPLE 14

| **Parenteral Formulation (IV)** | |
|---|---|
| The composition contains: | % wt./wt. |
| Active ingredient | 2.00% |
| Propylene glycol | 20.0% |
| Polyethylene glycol 400 | 20.0% |
| Polysorbate 80 | 1.0% |
| 0.9% Saline solution qs ad | 100 ml |

The active ingredient is dissolved in propylene glycol, polyethylene glycol 400 and polysorbate 80. A sufficient quantity of 0.9% saline solution is then added with stirring to provide 100 ml. of the I.V. solution which is filtered through a 0.2 micron membrane filter and packaged under sterile conditions.

### EXAMPLE 15

| **Suppository Formulation** | |
|---|---|
| The composition contains: | % wt./wt. |
| Active ingredient | 5.0% |
| Witepsol W | 95.0% |

The ingredients are melted together and mixed on a steam bath, and poured into molds containing 1-2 g total weight.

### EXAMPLE 16

| **Topical Formulation** | |
|---|---|
| Ingredients | grams |
| Active compound | 5-10 |
| Span 60 | 2 |
| Tween 60 | 2 |
| Mineral oil | 5 |
| Petrolatum | 10 |
| Methyl paraben | 0.15 |
| Propyl paraben | 0.05 |
| BHA (butylated hydroxy anisole) | 0.01 |
| Water | q.s. 100 |

All of the above ingredients, except water, are combined and heated to 60°C with stirring. A sufficient quantity of water at 60°C is then added with vigorous stirring to emulsify the ingredients, and water then added q.s. 100 g.

### EXAMPLE 17

### Determination of Affinity for Angiotensin II Receptors

Membranes of rat liver cells were homogenized (using a Polytron P10 tissue disrupter, setting 10 for 5 or 10 sec bursts) in 10 volumes (w/v) Tris buffer (pH 7.4 at 4°C) of the following composition: Tris HCl (50 mM) and Na₂ EDTA (5 mM). The homogenate was centrifuged at 500 x g and the supernatant retained. This procedure was repeated. The supernatant was rehomogenized and centrifuged at 30,000 to 48,000 x g, and the pellet resuspended in homogenizing buffer. Non-specific binding was determined using human angiotensin II (1.0 µM). AT¹ and AT² binding sites were labelled with [¹²⁵I]-Sar¹ Ile⁸ AII (0.01 nM; New England Nuclear). Incubations of test compound, radiolabel, and AT¹ or AT² binding sites were terminated by vacuum filtration over Whatman GF/B glass fiber filters. After filtration, the filters were washed, dried and counted for radioactivity by liquid scintillation spectrometry with quench correction.

The concentration of test compound producing 50% inhibition of specific radioligand binding was determined by iterative curve fitting and its inhibition dissociation constant (kᵢ) calculated.

1-(n-Butyl)-2-[2"-(1H-tetrazol-5-yl)biphenyl-4'-ylmethyl]indole-3-carboxylic acid was found active in this assay and has a pKi value of 7.7.

### EXAMPLE 18

### ANGIOTENSIN RECEPTOR ANTAGONISM FUNCTIONAL ASSAYS

(a) Isolated ring segments of rabbit aorta were set up in Krebs' physiological salt solution at 37°C, pH 7.4, under 1 g resting tension. The segments ware exposed to 50 mM KCl to evoke contraction. After thorough washing, cumulative concentration-effect curves to angiotensin II were constructed. Then, test compounds were preincubated with the tissue and a second concentration-effect curve to angiotensin II was constructed. A dextral shift in the concentration-effect curve to angiotensin II, greater than that seen in appropriate time controls, was taken as antagonistic activity of the test compound.
   The compounds of the present invention were active as antagonists of angiotensin II-mediated contraction in this assay.
(b) Isolated segments of guinea pig ileum were set up in Krebs' physiological salt solution at 37°C, pH 7.4, under 1 g resting tension. Non-cumulative concentration-effect curves to angiotensin II were constructed using 0.5 log unit increases in concentration. Then, test compounds were preincubated with the tissue and a second concentration-effect curve to angiotensin II was constructed. A dextral shift in the concentration-effect curve to angiotensin II, greater than that seen in appropriate time controls, was taken as antagonistic activity of the test compound.
   1-(n-Butyl)-2-[2"-(1H-tetrazol-5-yl)biphenyl-4'-ylmethyl]indole-3-carboxylic acid was found active as an antagonist in this assay.

### EXAMPLE 19

### Determination of Antihypertensive Activity

(a) Male normotensive rats were subjected to complete left renal artery ligation. Four to 8 days post ligation, systemic blood pressure and heart rate were recorded via the left femoral artery from conscious rats housed in Bollman restraint cages. Test and control compounds were administered via the femoral vein. The rats were divided into test and control groups.
   For intravenous administration, the test group received the test compound (in a vehicle of Tween®80 [polyoxyethylene sorbitan monooleate] and normal saline) in a dose of 10 or 30 mg/kg, whereas the control group received the appropriate volume of vehicle. Blood pressure and heart rate were monitored for 4 hours, at which time captopril (3 mg/kg, iv) was administered to both groups as a positive control, and blood pressure and heart rate were recorded for a further 30 minutes.
   For oral administration, the test group received a dose of 10 or 30 mg/Kg of the test compound, whereas the control group received the appropriate volume of vehicle. Blood pressure and heart rate were monitored for 4 hours, at which time captopril (3 mg/kg, iv) was administsred to both groups as a positive control and the same parameters were recorded for a further 30 minutes.
   The compounds of Formula (I), significantly lowered mean blood pressure when given by both the intravenous and oral routes.
(b) Normotensive rats were set-up for recording systemic blood pressure and heart rate as described for renal hypertensive rats in (a) above. Angiotensin II (0.1 µg/kg, iv) was administered intravenously to both control and test groups at 15 and 30 minutes prior to administration of vehicle or test compound, which was administered by bolus injection or infusion Angiotensin II (0.1 µg/kg, iv) was then injected every 30 minutes for 4 hours. A final dose of angiotensin II (1.0 µg/kg, iv) was given at the end of the experiment.
   The compounds of the present invention inhibited significantly the pressor response to angiotensin II without causing sustained hypotension, demonstrating that the compounds possess angiotensin II blocking properties in vivo and that their hypotensive action is selective to angiotensin II-dependent hypotension. (The systemic blood pressure of normotensive rats is not angiotensin II-dependent.)
   1-(n-Butyl)-2-[2"-(1H-tetrazol-5-yl)biphenyl-4'-ylmethyl]indole-3-carboxylic acid significantly blocked the pressor response to angiotensin II at an IV dose of 30 mg/Kg for 30 minutes.

### EXAMPLE 20

### COGNITIVE ENHANCEMENT ASSAY

The following describes a model to determine the cognitive enhancing effects of compounds of Formulae (I), (II) and (III).

Sprague Dawley rats (240-260 g) were kept in the laboratory the night prior to testing, and remained there throughout the experiment. The Morris Water Maze consists of a circular pool made from black perspex (122 cm diameter, 46 cm in height, with a 15 cm rim), filled with opaque water to a height of 35 cm. A hidden platform consisting of black plexiglass was placed 1-2 cm below the surface of the water. The pool was divided into four quadrants, arbitrarily corresponding to north, south, east and west. The platform was located in the south quadrant, about 24 cm from the side. Objects of high contrast were placed about the room to serve as spatial cues. A TV camera tracked the swim path of the rats, and the data thus obtained was examined to determine the time in seconds the rats took to find the platform (escape latency). Test trials were initiated by placing a rat into one of the four quadrants, facing the wall. Testing consisted of a block of six trials (starting first in the north quadrant, then east, south, west, north, and finally east) on each of two consecutive days. During each trial the rat was allowed 90 seconds to find the platform. When the rat successfully found the platform, it was given 30 seconds to "study" the spatial cues. When the rat failed to find the platform within 90 seconds, it was given a score of 90 seconds, and placed on the platform for 30 seconds.

The following groups of 8 rats each were used: 1) vehicle-treated controls; 2) atropine treated-controls; 3) atropine plus test drug. Thus the studies were designed to determine whether the test drug could alleviate the cognitive deficit induced by atropine (30 mg/kg, ip). Statistical tests were applied to test for heterogeneity of the learning curves, and separation of the learning curves.

1-(n-Butyl)-2-[2"-(1H-tetrazol-5-yl)biphenyl-4'-ylmethyl]indole-3-carboxylic acid was active in this assay at 0.01 to 10 mg/Kg i.p..

### EXAMPLE 21

### Toxicity

Rats were administered single oral doses of 30, 100 or 300 mg/Kg of 1-(n-butyl)-2-[2"-(1H-tetrazol-5-yl]biphenyl-4'-ylmethyl]indole-3-carboxylic acid and observed for 3 days postdosing. No mortality occurred at any of the doses given.

While the present invention has been described with respect to specific embodiments thereof, it will be understood by those skilled in the art that various changes may be made and equivalents may be substituted without departing from the true spirit and scope of the invention. All such modifications are intended to be within the scope of the claims appended hereto.

## Claims

1. A compound represented by the Formula (I) 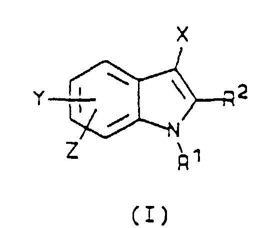 wherein:
R¹ is (C₁-C₁₀)alkyl (C₃-C₈)cycloalkyl or (C₃-C₈)cycloalkyl(C₁-C₁₀)alkyl;
R² is 2"-(1H-tetrazol-5-yl)biphenyl-4'-ylmethyl;
X is hydrogen, (C₁-C₁₀)alkyl, halogen, -C(O)CF₃, -CO₂R⁴, or -C(O)NR⁵R⁶;
Y is hydrogen, (C₁-C₁₀)alkyl, (C₁-C₁₀)alkoxy, hydroxy, halogen, or -CO₂R⁴;
Z is hydrogen, (C₁-C₁₀)alkyl, (C₁-C₁₀)alkoxy, or halogen;
in which
R⁴ is hydrogen or (C₁-C₁₀)alkyl;
R⁵ is hydrogen, (C₁-C₁₀)alkyl or -CH₂CO₂R⁴;
R⁶ is hydrogen or (C₁-C₁₀)alkyl; or
R⁵ and R⁶ taken together with the nitrogen to which they are attached represent a heterocycle selected from azetidine, azolidine, piperidine, 4-methylpiperidine, hexamethyleneimine, heptamethyleneimine;
or the pharmaceutically acceptable salts thereof.

2. The compound of claim 1 wherein X is -CO₂R⁴, or a pharmaceutically acceptable salt thereof.

3. The compound of Claim 2 wherein (i) R¹ is n-butyl, R⁴ is hydrogen, and Y and Z are hydrogen, namely 1-(n-butyl)-2-[2"-(1H-tetrazol-5-yl)biphenyl-4'-ylmethyl]indole-3-carboxylic acid, or a pharmaceutically acceptable salt thereof; or,
(ii) wherein R¹ is methyl, ethyl, n-propyl, isopropyl, n-pentyl, isopentyl, n-octyl, cyclopropylmethyl or cyclohexylmethyl, and R⁴ is hydrogen, and Y and Z are hydrogen namely respectively
1-methyl-2-[2"-(1H-tetrazol-5-yl)biphenyl-4'-ylmethyl]indole-3-carboxylic acid,
1-ethyl-2-[2"-(1H-tetrazol-5-yl)biphenyl-4'-ylmethyl]indole-3-carboxylic acid,
1-n-propyl-2-[2"-(1H-tetrazol-5-yl)biphenyl-4'-ylmethyl]indole-3-carboxylic acid,
1-isopropyl-2-[2"-(1H-tetrazol-5-yl)biphenyl-4'-yimethyl]indole-3-carboxylic acid,
1-n-pentyl-2-[2"-(1H-tetrazol-5-yl)biphenyl-4'-ylmethyl]indole-3-carboxylic acid, or
1-isopentyl-2-[2"-(1H-tetrazol-5-yl)biphenyl-4'-ylmethyl]indole-3-carboxylic acid, or
1-n-octyl-2-[2"-(1H-tetrazol-5-yl)biphenyl-4'-ylmethyl]indole-3-carboxylic acid,
1-cyclopropylmethyl-2-[2"-(1H-tetrazol-5-yl)biphenyl-4'-ylmethyl]indole-3-carboxylic acid, or
1-cyclohexylmethyl-2-[2"-(1H-tetrazol-5-yl)biphenyl-4'-ylmethyl)indole-3-carboxylic acid, or a pharmaceutically acceptable salt thereof; or,
(iii) wherein Y is 5-methyl or 7-methyl, R¹ is n-butyl and R⁴ is hydrogen, Z is hydrogen namely respectively
1-n-butyl-2-[2"-(1H-tetrazol-5-yl)biphenyl-4'-ylmethyl]-5-methylindole-3-carboxylic acid, or
1-n-butyl-2-[2"-(1H-tetrazol-5-yl)biphenyl-4'-ylmethyl]-7-methylindole-3-carboxylic acid, or a pharmaceutically acceptable salt thereof; or,
(iv) wherein Y is 5-methoxy or 6-methoxy, R¹ is n-butyl or cyclopropylmethyl and R⁴ is hydrogen, Z is hydrogen, namely
1-n-butyl-2-[2"-[1H-tetrazol-5-yl)biphenyl-4'-ylmethyl]-5-methoxyindole-3-carboxylic acid, or
1-n-butyl-2-[2"-(1H-tetrazol-5-yl)biphenyl-4'-ylmethyl)-6-methaxyindole-3-carboxylic acid, or
1-cyclopropylmethyl-2-(2"-(1H-tetrazol-5-yl)biphenyl-4'-ylmethyl]-6-methoxyindole-3-carboxylic acid, or a pharmaceutically acceptable salt thereof; or,
(v) wherein Y is 5-fluoro or chloro, R¹ is n-butyl, cyclopropylmethyl or cyclohexylmethyl and R⁴ is hydrogen, Z is hydrogen, namely
1-n-butyl-2-[2"-(1H-tetrazol-5-yl)biphenyl-4'-ylmethyl]-5-fluoroindole-3-carboxylic acid, or
1-n-butyl-2-[2"-(1H-tetrazol-5-yl)biphenyl-4'-ylmethyl]-5-chloroindole-3-carboxylic acid, or
1-cyclopropylmethyl-2-[2"-(1H-tetrazol-5-yl)biphenyl-4'-ylmethyl]-5-fluoroindole-3-carboxylic acid, or
1-cyclopropylmethyl-2-[2"-(1H-tetrazol-5-yl)biphenyl-4'-ylmethyl}-5-chloroindole-3-carboxylic acid, or
1-cyclohexylmethyl-2-[2"-(1H-tetrazol-5-yl)biphenyl-4'-ylmethyl]-5-fluoroindole-3-carboxylic acid, or
1-cyclohexylmethyl-2-[2"-(1H-tetrazol-5-yl)biphenyl-4'-ylmethyl]-5-chloroindole-3-carboxylic acid, or a pharmaceutically acceptable salt thereof.

4. The compound of Claim 1 wherein X is C(O)CF₃, R¹ is n-butyl, Y is hydrogen or 5-methoxy, Z is hydrogen namely
1-n-butyl-2-[2"-(1H-tetrazol-5-yl)biphenyl-4'-ylmethyl]-3-trifluoroacetylindole, or
1-n-butyl-5-methoxy-2-[2"-(1H-tetrazol-5-yl]biphenyl-4'-ylmethyl]-3-trifluoroacetylindole, or a pharmaceutically acceptable salt thereof.

5. The compound of Claim 1 wherein Y and Z are hydrogen and X is -C(O)NR⁵R⁶ wherein R⁵ is -CH₂CO₂R⁴, R⁶ is hydrogen and R⁴ is hydrogen or methyl, or a pharmaceutically acceptable salt thereof.

6. The compound of Claim 5 wherein R¹ is n-butyl, namely
1-n-butyl-2-[2"-(1H-tetrazol-5-yl)biphenyl-4'-ylmethyl]indole-3-carbonylaminoacetic acid, or
1-n-butyl-2-[2"-(1H-tetrazol-5-yl)biphenyl-4'-ylmethyl]-indole-3-carbonylammoacetic acid methyl ester, or a pharmaceutically accepable salt thereof.

7. A pharmaceutical composition comprising a therapeutically effective amount of a compound of any one of the preceding claims, or a pharmaceutically acceptable salt thereof, in admixture with one or more pharmaceutically acceptable non-toxic carriers.

8. The compound of anyone of claims 1 to 6 for use in a method of medical treatment.

9. Use of the compound of any one of claims 1 to 6 in the preparation of a medicament for treatment of a disease state alleviable with an angiotensin II antagonist.

10. The use of claim 9, wherein the disease state is hypertension or congestive heart failure, chronic renal failure or a disorder of the alimentary tract, or a cognitive disorder, anxiety or depression.

11. A process for preparing compounds of the Formula (I) 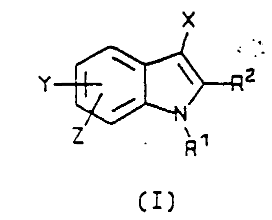 wherein:
R¹ is (C₁-C₁₀)alkyl, (C₃-C₈)cycloalkyl or (C₃-C₈)cycloalkyl (C₁-C₁₀)alkyl;
R² is 2"-(1H-tetrazol-5-yl)biphenyl-4'-ylmethyl;
X is hydrogen, (C₁-C₁₀) alkyl, halogen, -C(O)CF₃, -CO₃R⁴, or-C(O)NR⁵R⁶;
Y is hydrogen, (C₁-C₁₀)alkyl, (C₁-C₁₀)alkoxy, hydroxy, halogen, or -CO₂R⁴ ;
Z is hydrogen, (C₁-C₁₀)alkyl, (C₁-C₁₀)alkoxy, or halogen;
in which
R⁴ is hydrogen or (C₁-C₁₀)alkyl;
R⁵ is hydrogen, (C₁-C₁₀)alkyl or -CH₂CO₂R⁴;
R⁶ is hydrogen or (C₁-C₁₀)alkyl; or
R⁵ and R⁶ taken together with the nitrogen to which they are attached represent a heterocycle selected from azetidine, azolidine, piperidine 4-methylpiperidine, hexamethyleneimine, heptamethylenimine;
and the pharmaceutically acceptable salts thereof, comprising
(a) reacting a compound of Formula (1): 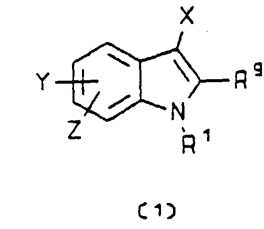 wherein X, Y, and Z are as defined above; R¹ is (C₁-C₁₀)alkyl, (C₃-C₈)cycloalkyl or (C₃-C₈)cycloalkyl(C₁-C₁₀)alkyl and R⁹ is 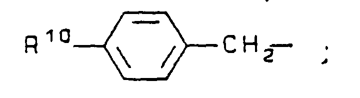 where R¹⁰ is: 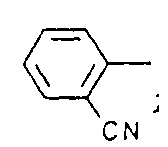 with a trialkyl tin azide followed by treatment with an acid to obtain a compound of Formula (I) wherein R¹, R², R⁴, R⁵, R⁶, R⁷, X, Y and Z are as defined above; or
(b) reacting a compound of Formula (1) with a base to obtain a compound of Formula (I) wherein R¹, R², R⁴, R⁵, R⁶, R⁷, X, Y and Z are as defined above; or
(c) reacting a compound of Formula (I) wherein R¹, R², R⁷, R⁸, Y and Z are as defined above and X is hydrogen, with phosgene optionally followed by an alcohol of formula R⁴OH, where R⁴ is lower alkyl, or an amine of formula R⁵R⁶NH, where R⁵ and R⁶ are as defined above, to form a compound of Formula (I) wherein R¹, R^{2,}R^{3,} R⁷, R⁸, Y, Z, V and W are as defined above, X is -CO₂R⁴ or -C(O)NR⁵R⁶ and R⁴ is (C₁-C₁₀)alkyl and R⁵ and R⁶ are as defined above; or
(d) reacting the compound of Formula (I) with a base to give a pharmaceutically acceptable base addition salt; or
(e) reacting a base addition salt of a compound of Formula (I) with an acid to give the corresponding free acid; or
(f) converting a base addition salt of a compound of Formula (I) to another pharmaceutically acceptable base addition salt of Formula (I).

12. A compound of Formula (1) : 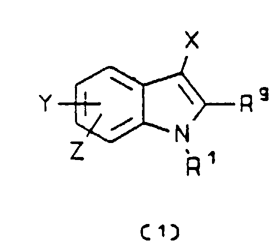 wherein X, Y, and Z are as defined above; R¹ is (C₁-C₁₀)alkyl, (C₃-C₈)cycloalkyl or (C₃-C₈)cycloalkyl(C₁-C₁₀)alkyl R⁹ is: 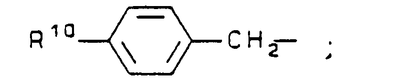 where R¹⁰ is:

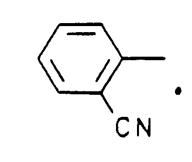

## Patentansprüche

1. Verbindung, die durch die Formel (I) dargestellt wird: 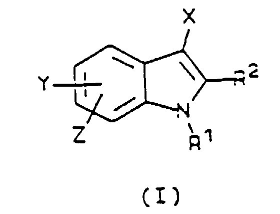 worin:
R¹ für (C₁-C₁₀)-Alkyl, (C₃-C₈)-Cycloalkyl oder (C₃-C₈)-Cycloalkyl-(C₁-C₁₀)-alkyl steht;
R² für 2"-(1H-Tetrazol-5-yl)biphenyl-4'-ylmethyl steht;
X Wasserstoff, (C₁-C₁₀)-Alkyl, Halogen, -C(O)CF₃, -CO₂R⁴ oder -C(O)NR⁵R⁶ ist;
Y Wasserstoff, (C₁-C₁₀)-Alkyl, (C₁-C₁₀)-Alkoxy, Hydroxy, Halogen oder -CO₂R⁴ ist;
Z Wasserstoff, (C₁-C₁₀)-Alkyl, (C₁-C₁₀)-Alkoxy oder Halogen ist;
wobei
R⁴ Wasserstoff oder (C₁-C₁₀)-Alkyl bedeutet;
R⁵ Wasserstoff, (C₁-C₁₀)-Alkyl oder -CH₂CO₂R⁴ bedeutet;
R⁶ Wasserstoff oder (C₁-C₁₀)-Alkyl bedeutet; oder
R⁵ und R⁶ mit dem Stickstoff, an den sie geknüpft sind, zusammengenommen einen aus Azetidin, Azolidin, Piperidin, 4-Methylpiperidin, Hexamethylenimin, Heptamethylenimin ausgewählten Heterozyklus darstellen;
oder die pharmazeutisch annehmbaren Salze derselben.

2. Verbindung nach Anspruch 1, in welcher X für -CO₂R⁴ steht, oder ein pharmazeutisch annehmbares Salz derselben.

3. Verbindung nach Anspruch 2, in welcher
(i) R¹ n-Butyl ist, R⁴ Wasserstoff ist und sowohl Y als auch Z Wasserstoff sind, d.h., 1-(n-Butyl)-2-[2"-(1H-tetrazol-5-yl)biphenyl-4'-ylmethyl]indol-3-carbonsäure, oder ein pharmazeutisch annehmbares Salz derselben; oder
(ii) in welcher R¹ Methyl, Ethyl, n-Propyl, Isopropyl, n-Pentyl, Isopentyl, n-Octyl, Cyclopropylmethyl oder Cyclohexylmethyl ist, sowohl Y als auch Z Wasserstoff sind und R⁴ Wasserstoff bedeutet, d.h.,
1-Methyl-2-[2"-(1H-tetrazol-5-yl)biphenyl-4'-ylmethyl]indol-3-carbonsäure,
1-Ethyl-2-[2"-(1H-tetrazol-5-yl)biphenyl-4'-ylmethyl]indol-3-carbonsäure,
1-n-Propyl-2-[2"-(1H-tetrazol-5-yl)biphenyl-4'-ylmethyl]indol-3-carbonsäure,
1-Isopropyl-2-[2"-(1H-tetrazol-5-yl)biphenyl-4'-ylmethyl]indol-3-carbonsäure,
1-n-Pentyl-2-[2"-(1H-tetrazol-5-yl)biphenyl-4'-ylmethyl]indol-3-carbonsäure,
1-Isopentyl-2-[2"-(1H-tetrazol-5-yl)biphenyl-4'-ylmethyl]indol-3-carbonsäure,
1-n-Octyl-2-[2"-(1H-tetrazol-5-yl)biphenyl-4'-ylmethyl]indol-3-carbonsäure,
1-Cyclopropylmethyl-2-[2"-(1H-tetrazol-5-yl)biphenyl-4'-ylmethyl]indol-3-carbonsäure, bzw.
1-Cyclohexylmethyl-2-[2"-(1H-tetrazol-5-yl)biphenyl-4'-ylmethyl]indol-3-carbonsäure, oder ein pharmazeutisch annehmbares Salz derselben; oder
(iii) in welcher Z Wasserstoff ist, Y für 5-Methyl oder 7-Methyl steht, R¹ n-Butyl bedeutet und R⁴ Wasserstoff darstellt, d.h.,
1-n-Butyl-2-[2"-(1H-tetrazol-5-yl)biphenyl-4'-ylmethyl]-5-methylindol-3-carbonsäure oder
1-n-Butyl-2-[2"-(1H-tetrazol-5-yl)biphenyl-4'-ylmethyl]-7-methylindol-3-carbonsäure, oder ein pharmazeutisch annehmbares Salz derselben; oder
(iv) in welcher Z Wasserstoff ist, Y für 5-Methoxy oder 6-Methoxy steht, R¹ n-Butyl oder Cyclopropylmethyl bedeutet und R⁴ Wasserstoff darstellt, d.h.,
1-n-Butyl-2-[2"-(1H-tetrazol-5-yl)biphenyl-4'-ylmethyl]-5-methoxyindol-3-carbonsäure oder
1-n-Butyl-2-[2"-(1H-tetrazol-5-yl)biphenyl-4'-ylmethyl]-6-methoxyindol-3-carbonsäure oder
1-Cyclopropylmethyl-2-[2"-(1 H-tetrazol-5-yl)biphenyl-4'-ylmethyl]-6-methoxyindol-3-carbonsäure, oder ein pharmazeutisch annehmbares Salz derselben; oder
(v) in welcher Z Wasserstoff ist, Y für 5-Fluor oder -Chlor steht, R¹ n-Butyl, Cyclopropylmethyl oder Cyclohexylmethyl bedeutet und R⁴ Wasserstoff darstellt, d.h.,
1-n-Butyl-2-[2"-(1H-tetrazol-5-yl)biphenyl-4'-ylmethyl]-5-fluorindol-3-carbonsäure oder
1-n-Butyl-2-[2"-(1H-tetrazol-5-yl)biphenyl-4'-ylmethyl]-5-chlorindol-3-carbonsäure oder
1-Cyclopropylmethyl-2-[2"-(1H-tetrazol-5-yl)biphenyl-4'-ylmethyl]-5-fluorindol-3-carbonsäure oder
1-Cyclopropylmethyl-2-[2"-(1H-tetrazol-5-yl)biphenyl-4'-ylmethyl]-5-chlorindol-3-carbonsäure oder
1-Cyclohexylmethyl-2-[2"-(1H-tetrazol-5-yl)biphenyl-4'-ylmethyl]-5-fluorindol-3-carbonsäure oder
1-Cyclohexylmethyl-2-[2"-(1H-tetrazol-5-yl)biphenyl-4'-ylmethyl]-5-chlorindol-3-carbonsäure, oder ein pharmazeutisch annehmbares Salz derselben.

4. Verbindung nach Anspruch 1, in welcher X für C(O)CF₃ steht, Z Wasserstoff ist, R¹ n-Butyl bedeutet, Y Wasserstoff oder 5-Methoxy darstellt, d.h.,
1-n-Butyl-2-[2"-(1H-tetrazol-5-yl)biphenyl-4'-ylmethyl]-3-trifluoracetylindol oder
1-n-Butyl-5-methoxy-2-[2"-(1H-tetrazol-5-yl)biphenyl-4'-ylmethyl]-3-trifluoracetylindol, oder ein pharmazeutisch annehmbares Salz derselben.

5. Verbindung nach Anspruch 1, in welcher Y und Z Wasserstoff darstellen und X für -C(O)NR⁵R⁶ steht, wobei R⁵ -CH₂CO₂R⁴ bedeutet, R⁶ Wasserstoff ist und R⁴ Wasserstoff oder Methyl darstellt, oder ein pharmazeutisch annehmbares Salz derselben.

6. Verbindung nach Anspruch 5, in welcher R¹ n-Butyl ist, d.h.,
1-n-Butyl-2-[2"-(1H-tetrazol-5-yl)biphenyl-4'-ylmethyl]-indol-3-carbonylaminoessigsäure oder
1-n-Butyl-2-[2"-(1H-tetrazol-5-yl)biphenyl-4'-ylmethyl]-indol-3-carbonylaminoessigsäuremethylester, oder ein pharmazeutisch annehmbares Salz derselben.

7. Pharmazeutische Zusammensetzung, umfassend eine therapeutisch wirksame Menge einer Verbindung nach irgendeinem der vorangehenden Ansprüche oder ein pharmazeutisch annehmbares Salz derselben in Mischung mit einem oder mehreren pharmazeutisch annehmbaren nichttoxischen Trägern.

8. Verbindung nach irgendeinem der Ansprüche 1 bis 6 zur Verwendung in einem Verfahren zur medizinischen Behandlung.

9. Verwendung der Verbindung nach irgendeinem der Ansprüche 1 bis 6 bei der Herstellung eines Medikaments zur Behandlung eines Krankheitszustands, der mit einem Angiotensin II-Antagonisten abgemildert werden kann.

10. Verwendung nach Anspruch 9, in welcher der Krankheitszustand Hochdruck oder Stauungs-Herzversagen, chronisches Nierenversagen oder eine Störung des Ernährungstrakts oder eine Wahrnehmungsstörung, Angst oder Depression ist.

11. Verfahren zur Herstellung von Verbindungen der Formel (I) 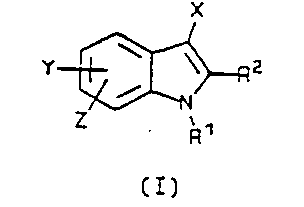 worin:
R¹ für (C₁-C₁₀)-Alkyl, (C₃-C₈)-Cycloalkyl oder (C₃-C₈)-Cycloalkyl-(C₁-C₁₀)-alkyl steht;
R² für 2"-(1H-Tetrazol-5-yl)biphenyl-4'-ylmethyl steht;
X Wasserstoff, (C₁-C₁₀)-Alkyl, Halogen, -C(O)CF₃, -CO₂R⁴ oder -C(O)NR⁵R⁶ ist;
Y Wasserstoff, (C₁-C₁₀)-Alkyl, (C₁-C₁₀)-Alkoxy, Hydroxy, Halogen oder -CO₂R⁴ ist;
Z Wasserstoff, (C₁-C₁₀)-Alkyl, (C₁-C₁₀)-Alkoxy oder Halogen ist;
wobei
gegebenenfalls gefolgt von einem Alkohol der Formel R⁴OH, worin R⁴ Niederalkyl bedeutet, oder einem Amin der Formel R⁵R⁶NH, worin R⁵ und R⁶ wie oben definiert sind, um eine Verbindung der Formel (I) zu bilden, in welcher R¹, R², R³, Y und Z wie oben definiert sind, X für -CO₂R⁴ oder -C(O)NR⁵R⁶ steht und R⁴ (C₁-C₁₀)-Alkyl bedeutet und R⁵ und R⁶ wie oben definiert sind; oder
(d) die Umsetzung der Verbindung der Formel (I) mit einer Base, um ein pharmazeutisch annehmbares Basenadditions-Salz zu ergeben; oder
(e) die Umsetzung eines Basenadditions-Salzes einer Verbindung der Formel (I) mit einer Säure, um die entsprechende freie Säure zu liefern; oder
(f) die Umwandlung eines Basenadditions-Salzes einer Verbindung der Formel (I) in ein anderes pharmazeutisch annehmbares Basenadditions-Salz der Formel (I).

12. Verbindung der Formel (1) 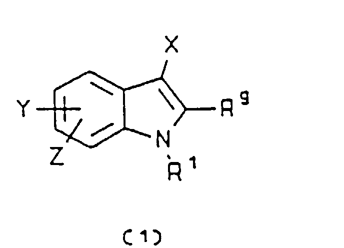 in welcher X, Y und Z wie oben definiert sind; R¹ (C₁-C₁₀)-Alkyl, (C₃-C₈)-Cycloalkyl oder (C₃-C₈)-Cycloalkyl-(C₁-C₁₀)-alkyl bedeutet und R⁹ für 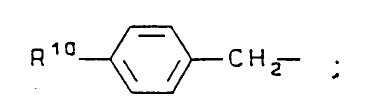 steht,
wobei R¹⁰ 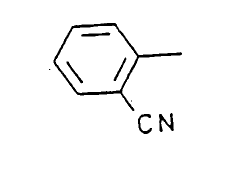 bedeutet.
R⁴ Wasserstoff oder (C₁-C₁₀)-Alkyl bedeutet;
R⁵ Wasserstoff, (C₁-C₁₀)-Alkyl oder -CH₂CO₂R⁴ bedeutet;
R⁶ Wasserstoff oder (C₁-C₁₀)-Alkyl bedeutet; oder
R⁵ und R⁶ mit dem Stickstoff, an den sie geknüpft sind, zusammengenommen einen aus Azetidin, Azolidin, Piperidin, 4-Methylpiperidin, Hexamethylenimin, Heptamethylenimin ausgewählten Heterozyklus darstellen;
und der pharmazeutisch annehmbaren Salze derselben, umfassend
(a) die Umsetzung einer Verbindung der Formel (1) 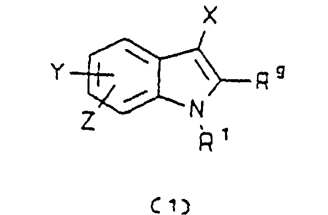 worin X, Y und Z wie oben definiert sind; R¹ für (C₁-C₁₀)-Alkyl, (C₃-C₈)-Cycloalkyl oder (C₃-C₈)-Cycloalkyl-(C₁-C₁₀)-alkyl steht und R⁹ 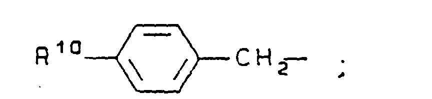 bedeutet;
wobei R¹⁰ 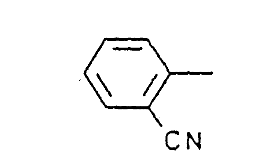 darstellt;
mit einem Trialkylzinnazid, gefolgt von der Behandlung mit einer Säure, um eine Verbindung der Formel (I) zu erhalten, in welcher R¹, R², R⁴, R⁵, R⁶, X, Y und Z wie oben definiert sind; oder
(b) die Umsetzung einer Verbindung der Formel (1) mit einer Base, um eine Verbindung der Formel (I) zu erhalten, in welcher R¹, R², R⁴, R⁵, R⁶, X, Y und Z wie oben definiert sind; oder
(c) die Umsetzung einer Verbindung der Formel (1) in welcher R¹, R², Y und Z wie oben definiert sind und X für Wasserstoff steht, mit Phosgen,

## Revendications

1. Composé représenté par la formule (I) 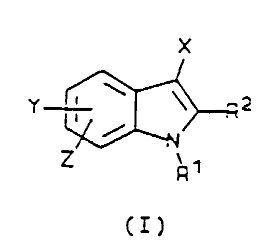 dans laquelle :
R¹ représente un groupe alkyle en C₁ à C₁₀, cycloalkyle en C₃ à C₈ ou (cycloalkyle en C₃ à C₈)-(alkyle en C₁ à C₁₀) ;
R² représente un groupe 2"-(1H-tétrazole-5-yl)biphényl-4'-ylméthyle ;
X représente l'hydrogène, un groupe alkyle en C₁ à C₁₀, halogéno, -C(O)-CF₃, -CO₂R⁴ ou -C(O)NR⁵R⁶ ;
Y représente l'hydrogène, un groupe alkyle en C₁ à C₁₀, alkoxy en C₁ à C₁₀, hydroxy, halogéno ou -CO₂R⁴ ;
Z représente l'hydrogène, un groupe alkyle en C₁ à C₁₀, alkoxy en C₁ à C₁₀ ou halogéno ;
groupes dans lesquels
R⁴ représente l'hydrogène ou un groupe alkyle en C₁ à C₁₀,
R⁵ représente l'hydrogène, un groupe alkyle en C₁ à C₁₀ ou -CH₂CO₂R⁴ ;
R⁶ représente l'hydrogène ou un groupe alkyle en C₁ à C₁₀ ; ou
R⁵ et R⁶, pris conjointement avec l'atome d'azote auquel ils sont fixés, représentent un hétérocycle choisi entre les hétérocycles azétidine, azolidine, pipéridine, 4-méthylpipéridine, hexaméthylène-imine et heptaméthylèneimine ;
ou ses sels pharmaceutiquement acceptables.

2. Composé suivant la revendication 1, dans lequel X représente un groupe -CO₂R⁴, ou un de ses sels pharmaceutiquement acceptables.

3. Composé suivant la revendication 2, dans lequel
(i) R¹ représente un groupe n-butyle, R⁴ représente l'hydrogène et Y et Z représentent l'un et l'autre l'hydrogène, à savoir l'acide 1-(n-butyl)-2-(2"-(1H-tétrazole-5-yl)biphényl-4'-ylméthyl)indole-3-carboxylique, ou un de ses sels pharmaceutiquement acceptables ; ou
(ii) R¹ représente un groupe méthyle, éthyle, n-propyle, isopropyle, n-pentyle, isopentyle, n-octyle, cyclopropylméthyle ou cyclohexylméthyle, Y et Z représentent l'un et l'autre l'hydrogène et R⁴ représente l'hydrogène, à savoir, respectivement,
l'acide 1-méthyl-2-[2"-(1H-tétrazole-5-yl)-biphényl-4'-ylméthyl]indole-3-carboxylique,
l'acide 1-éthyl-2-[2"-(1H-tétrazole-5-yl)biphényl-4'-ylméthyl]indole-3-carboxylique,
l'acide 1-n-propyl-2-[2"-(1H-tétrazole-5-yl)biphényl-4'-ylméthyl]indole-3-carboxylique,
l'acide 1-isopropyl-2-[2"-(1H-tétrazole-5-yl)biphényl-4'-ylméthyl]indole-3-carboxylique,
l'acide 1-n-pentyl-2-[2"-(1H-tétrazole-5-yl)biphényl-4'-ylméthyl]indole-3-carboxylique, ou
l'acide 1-isopentyl-2-[2"-(1H-tétrazole-5-yl)biphényl-4'-ylméthyl]indole-3-carboxylique, ou
l'acide 1-n-octyl-2-[2"-(1H-tétrazole-5-yl)biphényl-4'-ylméthyl]indole-3-carboxylique,
l'acide 1-cyclopropylméthyl-2-[2"-(1H-tétrazole-5-yl)biphényl-4'-ylméthyl]indole-3-carboxylique, ou
l'acide 1-cyclohexylméthyl-2-[2"-(1H-tétrazole-5-yl)biphényl-4'-ylméthyl]indole-3-carboxylique, ou un de ses sels pharmaceutiquement acceptables ; ou
(iii) Z représente l'hydrogène, Y représente un groupe 5-méthyle ou 7-méthyle, R¹ représente un groupe n-butyle et R⁴ représente l'hydrogène, à savoir, respectivement
l'acide 1-n-butyl-2-[2"-(1H-tétrazole-5-yl)biphényl-4'-ylméthyl]-5-méthylindole-3-carboxylique, ou
l'acide 1-n-butyl-2-[2"-(1H-tétrazole-5-yl)biphényl-4-ylméthyl)-7-méthylindole-3-carboxylique, ou un de ses sels pharmaceutiquement acceptables ; ou
(iv) Z représente l'hydrogène, Y représente un groupe 5-méthoxy ou 6-méthoxy, R¹ représente un groupe n-butyle ou cyclopropylméthyle et R⁴ représente l'hydrogène, à savoir
l'acide 1-n-butyl-2-[2"-(1H-tétrazole-5-yl)biphényl-4'-ylméthyl]-5-méthoxyindole-3-carboxylique, ou
l'acide 1-n-butyl-2-[2"-(1H-tétrazole-5-yl)biphényl-4'-ylméthyl]-6-méthoxyindole-3-carboxylique, ou
l'acide 1-cyclopropylméthyl-2-[2"-(1H-tétrazole-5-yl)biphényl-4'-ylméthyl]-6-méthoxyindole-3-carboxylique, ou un de ses sels pharmaceutiquement acceptables ; ou bien
(v) dans lequel Z représente l'hydrogène, Y représente un groupe 5-fluoro ou chloro, R¹ représente un groupe n-butyle, cyclopropylméthyle ou cyclohexylméthyle et R⁴ représente l'hydrogène, à savoir
l'acide 1-n-butyl-2-[2"-(1H-tétrazole-5-yl)biphényl-4'-ylméthyl]-5-fluoro-indole-3-carboxylique, ou
l'acide 1-n-butyl-2-[2"-(1H-tétrazole-5-yl)biphényl-4'-ylméthyl]-5-chloro-indole-3-carboxylique, ou
l'acide 1-cyclopropylméthyl-2-[2"-(1H-tétrazole-5-yl)biphényl-4'-ylméthyl]-5-fluoro-indole-3-carboxylique, ou
l'acide 1-cyclopropylméthyl-2-[2"-(1H-tétrazole-5-yl)biphényl-4'-ylméthyl]-5-chloro-indole-3-carboxylique, ou
l'acide 1-cyclohexylméthyl-2-[2"-(1H-tétrazole-5-yl)biphényl-4'-ylméthyl]-5-fluoro-indole-3-carboxylique, ou
l'acide 1-cyclohexylméthyl-2-[2"-(1H-tétrazole-5-yl)biphényl-4'-ylméthyl]-5-chloro-indole-3-carboxylique, ou un de ses sels pharmaceutiquement acceptables.

4. Composé suivant la revendication 1, dans lequel X représente un groupe C(O)CF₃, Z représente l'hydrogène, R¹ représente un groupe n-butyle, Y représente l'hydrogène ou un groupe 5-méthoxy, à savoir
le 1-n-butyl-2-[2"-(1H-tétrazole-5-yl)biphényl-4'-ylméthyl]-3-trifluoroacétylindole, ou
le 1-n-butyl-5-méthoxy-2-[2"-(1H-tétrazole-5-yl)biphényl-4'-ylméthyl]-3-trifluoracétylindole, ou un de ses sels pharmaceutiquement acceptables.

5. Composé suivant la revendication 1, dans lequel Y et Z représentent l'hydrogène et X représente un groupe -C(O)NR⁵R⁶ dans lequel R⁵ représente un groupe -CH₂CO₂R⁴, R⁶ représente l'hydrogène et R⁴ représente l'hydrogène ou un groupe méthyle, ou un de ses sels pharmaceutiquement acceptables.

6. Composé suivant la revendication 5, dans lequel R¹ représente un groupe n-butyle, à savoir
l'acide 1-n-butyl-2-[2"-(1H-tétrazole-5-yl)biphényl-4'-ylméthyl]-indole-3-carbonylaminoacétique, ou
l'ester méthylique d'acide 1-n-butyl-2-[2"-(1H-tétrazole-5-yl)biphényl-4'-ylméthyl]-indole-3-carbonyl-aminoacétique, ou un de ses sels pharmaceutiquement acceptables.

7. Composition pharmaceutique comprenant une quantité thérapeutiquement efficace d'un composé suivant l'une quelconque des revendications précédentes, ou d'un de ses sels pharmaceutiquement acceptables, en mélange avec un ou plusieurs supports non toxiques pharmaceutiquement acceptables.

8. Composé suivant l'une quelconque des revendications 1 à 6, destiné à être utilisé dans une méthode de traitement médicamenteux.

9. Utilisation du composé suivant l'une quelconque des revendications 1 à 6 dans la préparation d'un médicament destiné au traitement d'un état pathologique pouvant être soulagé avec un antagoniste d'angiotensine II.

10. Utilisation suivant la revendication 9, dans laquelle l'état pathologique consiste en hypertension ou insuffisance cardiaque congestive, insuffisance rénale chronique ou un trouble du tube digestif, ou un trouble cognitif, l'anxiété ou la dépression.

11. Procédé pour la préparation de composés de formule (I) 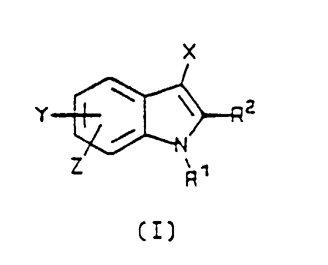 dans laquelle :
R¹ représente un groupe alkyle en C₁ à C₁₀, cycloalkyle en C₃ à C₈ ou (cycloalkyle en C₃ à C₈)-(alkyle en C₁ à C₁₀) ;
R² représente un groupe 2"-(1H-tétrazole-5-yl)biphényl-4'-ylméthyle ;
X représente l'hydrogène, un groupe alkyle en C₁ à C₁₀, halogéno, -C(O)-CF₃, -CO₂R⁴ ou -C(O)NR⁵R⁶ ;
Y représente l'hydrogène, un groupe alkyle en C₁ à C₁₀, alkoxy en C₁ à C₁₀, hydroxy, halogéno ou -CO₂R⁴ ;
Z représente l'hydrogène, un groupe alkyle en C₁ à C₁₀, alkoxy en C₁ à C₁₀ ou halogéno ;
groupes dans lesquels
R⁴ représente l'hydrogène ou un groupe alkyle en C₁ à C₁₀,
R⁵ représente l'hydrogène, un groupe alkyle en C₁ à C₁₀ ou -CH₂CO₂R⁴ ;
R⁶ représente l'hydrogène ou un groupe alkyle en C₁ à C₁₀ ; ou
R⁵ et R⁶, pris conjointement avec l'atome d'azote auquel ils sont fixés, représentent un hétérocycle choisi entre les hétérocycles azétidine, azolidine, pipéridine, 4-méthylpipéridine, hexaméthylène-imine et heptaméthylène-imine ;
et de leurs sels pharmaceutiquement acceptables, comprenant
(a) la réaction d'un composé de formule (1) 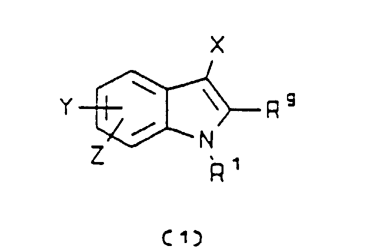 dans laquelle X, Y et Z répondent aux définitions précitées, R¹ représente un groupe alkyle en C₁ à C₁₀, cycloalkyle en C₃ à C₈ ou (cycloalkyle en C₃ à C₈)-(alkyle en C₁ à C₁₀) et R⁹ représente un groupe 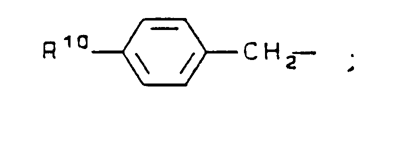 dans lequel R¹⁰ représente un groupe : 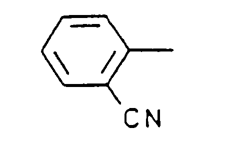 avec un azoture de trialkylétain, puis un traitement avec un acide pour obtenir un composé de formule (I) dans laquelle R¹, R², R⁴, R⁵, R⁶, X, Y et Z répondent aux définitions précitées ; ou
(b) la réaction d'un composé de formule (1) avec une base pour obtenir un composé de formule (I) dans laquelle R¹, R², R⁴, R⁵, R⁶, X, Y et Z répondent aux définitions précitées ; ou
c) la réaction d'un composé de formule (I) dans laquelle R¹, R², Y et Z répondent aux définitions précitées et X représente l'hydrogène, avec le phosgène suivi facultativement par un alcool de formule R⁴OH, dans laquelle R⁴ représente un groupe alkyle inférieur, ou par une amine de formule R⁵R⁶NH dans laquelle R⁵ et R⁶ répondent aux définitions précitées, pour former un composé de formule (I) dans laquelle R¹, R², R³, Y, Z répondent aux définitions précitées, X représente un groupe -CO₂R⁴ ou -C(O)NR⁵R⁶ et R⁴ représente un groupe alkyle en C₁ à C₁₀ et R⁵ et R⁶ répondent aux définitions précitées ; ou
(d) la réaction du composé de formule (I) avec une base, ce qui donne un sel d'addition de base pharmaceutiquement acceptable ; ou
(e) la réaction d'un sel d'addition de base d'un composé de formule (I) avec un acide, ce qui donne l'acide libre correspondant, ou
(f) la transformation d'un sel d'addition de base d'un composé de formule (I) en un autre sel d'addition de base pharmaceutiquement acceptable de formule (I).

12. Composé de formule (1) 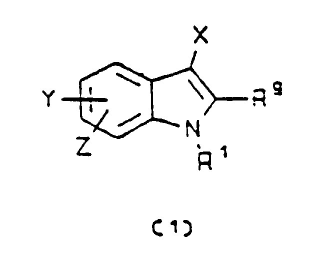 dans laquelle X, Y et Z répondent aux définitions précitées ; R¹ représente un groupe alkyle en C₁ à C₁₀, cycloalkyle en C₃ à C₈ ou (cycloalkyle en C₃ à C₈)-(alkyle en C₁ à C₁₀), R⁹ représente un groupe 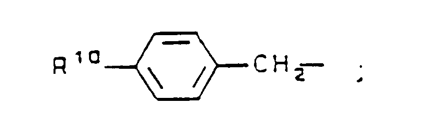 dans laquelle R¹⁰ représente un groupe :
